# FASCICULE DE BREVET EUROPEEN

(11) **EP 0 832 057 B1**
(45) Date de publication et mention de la délivrance du brevet: **03.01.2001**
(21) Numéro de dépôt: 97908307.8
(22) Date de dépôt: 05.03.1997
(51) Int. Cl.: C07C 63/66, A61K 31/19, C07C 313/04, C07C 317/22, C07C 65/19, C07C 65/40, C07C 69/78, C07D 335/06, C07D 333/38, C07D 207/40

(54) **COMPOSES PROPYNYL OU DIENYL BIAROMATIQUES**
DIAROMATISCHE PROPYNYL- ODER DIENYLVERBINDUNGEN
DIAROMATIC PROPYNYL OR DIENYL COMPOUNDS

(30) Priorité: 14.03.1996 FR 9603234
(43) Date de publication de la demande: 01.04.1998
(73) Titulaire: CENTRE INTERNATIONAL DE RECHERCHES DERMATOLOGIQUES GALDERMA (C.I.R.D. GALDERMA), F-06560 Valbonne (FR)
(72) Inventeur: BERNARDON, Jean-Michel, F-06650 Le Rouret (FR)
(74) Mandataire: Tezier Herman, Béatrice
(86) Numéro de dépôt international: FR9700390
(87) Numéro de publication internationale: WO9733856

(56) Documents cités:
- EP-A- 0 661 258

## Description

L'invention concerne, à titre de produits industriels nouveaux et utiles, des composés propynyl ou diényl biaromatiques. Elle concerne également l'utilisation de ces nouveaux composés dans des compositions pharmaceutiques destinées à un usage en médecine humaine ou vétérinaire, ou bien encore dans des compositions cosmétiques.

Le document EP-0661 258 (D1) décrit des composés propynyl bi-aromatiques qui peuvent présenter une structure de formule (I) dans laquelle l'un des substituants correspondant à R₈ ou R₉ représente un atome d'hydrogène ou un radical alkyle inférieur, mais qui diffèrent des composés de l'invention par l'autre substituant correspondant à R₈ ou R₉ qui représente un radical-OR₁₀.

Les composés selon l'invention ont une activité marquée dans les domaines de la différenciation et de la prolifération cellulaire, et trouvent des applications plus particulièrement dans le traitement topique et systémique des affections dermatologiques liées à un désordre de la kératinisation, des affections dermatologiques (ou autres) à composante inflammatoire et/ou immunoallergique, et des proliférations dermiques ou épidermiques qu'elles soient bénignes ou malignes. Ces composés peuvent en outre être utilisés dans le traitement des maladies de dégénérescence du tissu conjonctif, pour lutter contre le vieillissement de la peau, qu'il soit photoinduit ou chronologique, et traiter les troubles de la cicatrisation. Ils trouvent par ailleurs une application dans le domaine ophtalmologique, notamment dans le traitement des cornéopathies.

On peut également utiliser les composés selon l'invention dans des compositions cosmétiques pour l'hygiène corporelle et capillaire.

Les composés selon l'invention peuvent être représentés par la formule générale (I) suivante : 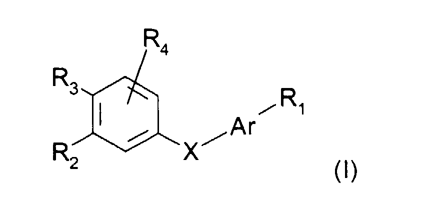 dans laquelle:
- R₁ représente
   (i) le radical -CH₃
   (ii) le radical -CH₂-O-R₆
   (iii) le radical -O-R₆
   (iv) le radical -CO-R₇
   R₆ et R₇ ayant les significations données ci-après,
- Ar représente un radical choisi parmi les radicaux de formules (a)-(e) suivantes: 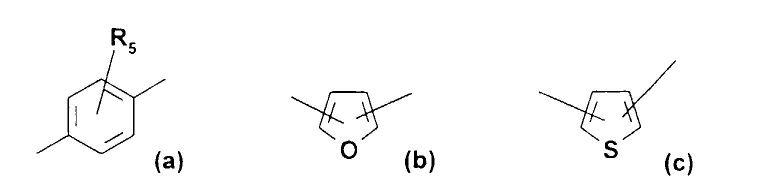 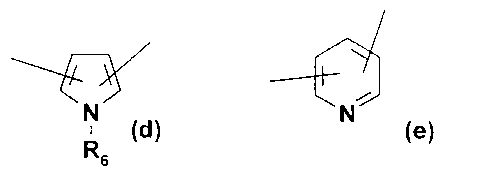 R₅ et R₆ ayant les significations données ci-après,
- X représente un radical de formule: 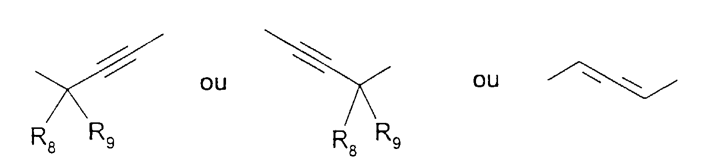 R₈ et R₉ ayant les significations données ci-après,
- R₂ et R₃, identiques ou différents, représentent
   (i) un atome d'hydrogène,
   (ii) un radical alkyle linéaire ou ramifié ayant de 1 à 20 atomes de carbone,
   (iii) un radical -OR_{6,}
   (iv) un radical -SR₆,
   R₆ ayant la signification donnée ci-après,
étant entendu que R₂ et R₃ pris ensemble peuvent former avec le cycle aromatique adjacent un cycle à 5 ou 6 chainons éventuellement substitué par des groupes méthyle et/ou éventuellement interrompu par un atome d'oxygène ou de soufre,
et étant entendu que R₂ et R₃ ne peuvent pas avoir en même temps les significations (i), (iii) et (iv) mentionnées ci-dessus.
- R₄ et R₅, identiques ou différents, représentent un atome d'hydrogène, un atome d'halogène, un radical alkyle linéaire ou ramifié ayant de 1 à 20 atomes de carbone, un radical -OR₆,
   étant entendu que lorsque R₄ est un radical hydroxyle alors R₂ et R₃ forment avec le cycle aromatique adjacent un cycle à 5 ou 6 chainons éventuellement substitué par des groupes méthyle et/ou éventuellement interrompu par un atome d'oxygène ou de soufre,
- R₆ représente un atome d'hydrogène, un radical alkyle inférieur ou un radical -COR₁₀
   R₁₀ ayant la signification donnée ci-après,
- R₇ représente:
   (a) un atome d'hydrogène
   (b) un radical alkyle inférieur
   (c) un radical de formule: 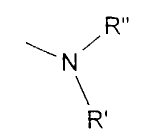
   R' et R" ayant la signification donnée ci-après,
   (d) un radical -OR₁₁,
   (e) un radical -NHOR₆,
   R₁₁ ayant la signification donnée ci-après,
- R₈ et R₉, pris séparément, soit ont simultanément la même signification : un atome d'hydrogène, soit l'un représente un atome d'hydrogène et l'autre un radical alkyle inférieur ou , pris ensemble, forment un cycle -Y-(CH₂)n-Y-, avec Y représentant un atome d'oxygène ou de soufre et avec n égal à 2 ou 3,
- R₁₀ représente un radical alkyle inférieur,
- R₁₁ représente un atome d'hydrogène, un radical alkyle linéaire ou ramifié, ayant de 1 à 20 atomes de carbone, un radical alkényl, un radical mono ou polyhydroxyalkyle, un radical aryle ou aralkyle, éventuellement substitué(s) ou un reste de sucre ou un reste d'amino acide ou de peptide,
- R' et R", identiques ou différents, représentent un atome d'hydrogène, un radical alkyl inférieur, un radical mono ou polyhydroxyalkyle, un radical aryle éventuellement substitué ou un reste d'amino acide ou de sucre ou encore pris ensemble forment un hétérocycle.

L'invention vise également les composés, qui sont des intermédiaires de synthèse des composés de formule générale (I), de formule générale (II) 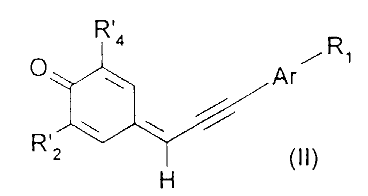 dans laquelle R₁ et Ar ont les mêmes significations que pour la formule générale (I) et R'₂ et R'₄, identiques ou différents, représentent un radical alkyle linéaire ou ramifié ayant de 1 à 20 atomes de carbone.

L'invention vise également les sels des composés de formules (I) et (II) lorsque R1 représente une fonction acide carboxylique et les isomères géométriques et optiques desdits composés de formules (I) et (II).

Lorsque les composés selon l'invention se présentent sous forme de sels, il s'agit de préférence de sels d'un métal alcalin ou alcalino-terreux, ou encore de zinc ou d'une amine organique.

Selon la présente invention, on entend par radical alkyle inférieur un radical ayant de 1 à 12, de préférence de 1 à 9, atomes de carbone, avantageusement les radicaux méthyle, éthyle, propyle, isopropyle, butyle, tertiobutyle, pentyle, hexyle, heptyle, nonyle, décyle et dodécyle.

Par radical alkyle linéaire ayant de 1 à 20 atomes de carbone, on entend notamment les radicaux méthyle, éthyle, propyle, pentyle, hexyle, octyle, décyle, dodécyle, hexadécyle et octadécyl.

Par radical alkyle ramifié ayant de 1 à 20 atomes de carbone, on entend notamment les radicaux 2-éthylhexyle, 2-méthylbutyle, 2-méthylpentyle, 1-méthylhexyle, 3-méthylheptyle.

Parmi les radicaux monohydroxyalkyle, on préfère un radical présentant 2 ou 3 atomes de carbone, notamment un radical 2-hydroxyéthyle, 2-hydroxypropyle ou 3-hydroxypropyle.

Parmi les radicaux polyhydroxyalkyle, on préfère un radical présentant de 3 à 6 atomes de carbone et de 2 à 5 groupes hydroxyles, tels que les radicaux 2,3-dihydroxypropyle, 2,3,4-trihydroxybutyle, 2,3,4,5-tétrahydroxypentyle ou le reste du pentaérythritol.

Parmi les radicaux aryle, on préfère un radical phényle éventuellement substitué par au moins un atome d'halogène, un hydroxyle ou une fonction nitro.

Parmi les radicaux aralkyle, on préfère le radical benzyle ou phénéthyle éventuellement substitué par au moins un atome d'halogène, un hydroxyle ou une fonction nitro.

Parmi les radicaux alkényle, on préfère un radical contenant de 2 à 5 atomes de carbone et présentant une ou plusieurs insaturations éthyléniques, tel que plus particulièrement le radical allyle.

Par reste de sucre, on entend un reste dérivant notamment de glucose, de galactose ou de mannose, ou bien encore de l'acide glucuronique.

Par reste d'aminoacide, on entend notamment un reste dérivant de la lysine, de la glycine ou de l'acide aspartique, et par reste de peptide on entend plus particulièrement un reste de dipeptide ou de tripeptide résultant de la combinaison d'acides aminés.

Par hétérocycle enfin, on entend de préférence un radical pipéridino, morpholino, pyrrolidino ou pipérazino, éventuellement substitué en position 4 par un radical alkyle en C₁-C₆ ou mono ou polyhydroxyalkyle tels que définis ci-dessus.

Lorsque les radicaux R₄ et R₅ représentent un atome d'halogène, celui-ci est de préférence un atome de fluor, de brome ou de chlore.

Parmi les composés de formule (I) ci-dessus rentrant dans le cadre de la présente invention, on peut notamment citer les composés suivants:
- Acide 4-[3-(3,5-di-tert-butyl-4-hydroxyphenyl)-1-propynyl] benzoïque.
- Acide 2-hydroxy-4-[3-(3,5-di-tert-butyl-4-hydroxyphenyl)-1-propynyl] benzoïque.
- Acide 4-[3-(5,6,7,8-tétrahydro-5,5,8,8-tétraméthyl-2-naphtyl)-1-propynyl] benzoïque.
- 2-hydroxy-4-[3-(5,6,7,8-tétrahydro-5,5,8,8-tétraméthyl-2-naphtyl)-1-propynyl]benzoate de méthyle.
- Acide 2-hydroxy-4-[3-(5,6,7,8-tétrahydro-5,5,8,8-tétraméthyl-2-naphtyl)-1-propynyl]benzoïque.
- Acide 2-hydroxy-4-[3-(3-hydroxy-5,6,7,8-tétrahydro-5,5,8,8-tétraméthyl-2-naphtyl)-1-propynyl]benzoïque.
- 2-hydroxy-4-[3-(5,6,7,8-tétrahydro-5,5,8,8-tétraméthyl-2-naphtyl)-1-propynyl] benzèneméthanol.
- 2-hydroxy-4-[3-(5,6,7,8-tétrahydro-5,5,8,8-tétraméthyl-2-naphtyl)-1-propynyl] benzoate de diéthanolamine.
- 2-hydroxy-4-[3-(5,6,7,8-tétrahydro-5,5,8,8-tétraméthyl-2-naphtyl)-1-propynyl] benzoate de lithium.
- Acide 4-[3-(5,6,7,8-tétrahydro-5,5,8,8-tétraméthyl-2-naphtyl)-2-propynyl] benzoïque.
- Acide 2-hydroxy-4-[3-(4,4-dimethylthiochromane-6-yl)-1-propynyl]benzoïque.
- Acide 2-hydroxy-4-[3-(8,8-diméthyl-5,6,7,8-tétrahydro-2-naphtyl)-1-propynyl] benzoïque.
- Acide 2-hydroxy-4-[3-(5,5-diméthyl-5,6,7,8-tétrahydro-2-naphtyl)-1-propynyl] benzoïque.
- 4-[3-(5,6,7,8-tétrahydro-5,5,8,8-tétraméthyl-2-naphtyl)-1-propynyl]benzoate d'ethyle.
- 4-[3-(5,6,7,8-tétrahydro-5,5,8,8-tétraméthyl-2-naphtyl)-1-propynyl] benzamide.
- N-ethyl-4-[3-(5,6,7,8-tétrahydro-5,5,8,8-tétraméthyl-2-naphtyl)-1-propynyl] benzamide.
- Morpholide de l'acide 4-[3-(5,6,7,8-tétrahydro-5,5,8,8-tétraméthyl-2-naphtyl)-1-propynyl]benzoïque.
- N-(4-hydroxyphenyl)-4-[3-(5,6,7,8-tétrahydro-5,5,8,8-tétraméthyl-2-naphtyl)-1-propynyl]benzamide
- 4-[3-(5,6,7,8-tétrahydro-5,5,8,8-tétraméthyl-2-naphtyl)-1-propynyl] benzaldehyde.
- 4-[3-(5,6,7,8-tétrahydro-5,5,8,8-tétraméthyl-2-naphtyl)-1-propynyl]phenol.
- [3-(5,6,7,8-tétrahydro-5,5,8,8-tétraméthyl-2-naphtyl)-1-propynyl]benzene methanol
- 4-[3-(5,6,7,8-tétrahydro-5,5,8,8-tétraméthyl-2-naphtyl)-1-propynyl]toluene.
- 4-[3-(5,6,7,8-tétrahydro-5,5,8,8-tétraméthyl-2-naphtyl)-1-propynyl]benzoate d'hexyle.
- N-hydroxy-4-[3-(5,6,7,8-tétrahydro-5,5,8,8-tétraméthyl-2-naphtyl)-1-propynyl] benzamide.
- N-hydroxy-2-hydroxy-4-[3-(5,6,7,8-tétrahydro-5,5,8,8-tétraméthyl-2-naphtyl)-1-propynyl]benzamide.
- Acide 2-methyl-4-[3-(5,6,7,8-tétrahydro-5,5,8,8-tétraméthyl-2-naphtyl)-1-propynyl]benzoïque.
- Acide 3-methyl-4-[3-(5,6,7,8-tétrahydro-5,5,8,8-tétraméthyl-2-naphtyl)-1-propynyl]benzoïque.
- Acide 6-[3-(5,5,8,8-tetramethyl-5,6,7,8-tetrahydro--2-naphtyl)propa-1,2-dienyl]nicotinique.
- Acide 4-[3-(5,5,8,8-tetramethyl-5,6,7,8-tetrahydro--2-naphtyl)propa-1,2-dienyl]benzoïque.
- Acide 2-hydroxy-4-[3-(5,5,8,8-tetramethyl-5,6,7,8-tetrahydro--2-naphtyl) propa-1,2-dienyl]benzoïque.
- Acide 2-hydroxy-4-[3-(5,6,7,8-tétrahydro-5,5,8,8-tétraméthyl-2-naphtyl)-1-butynyl]benzoïque.
- Acide 5-[3-(5,6,7,8-tétrahydro-5,5,8,8-tétraméthyl-2-naphtyl)-1-propynyl]-2-pyridinecarboxylique.
- Acide 4-[3-(3,5,5,8,8-pentaméthyl-5,6,7,8-tétrahydro-2-naphtyl)-1-propynyl] benzoïque.
- Acide 2-[3-(5,6,7,8-tétrahydro-5,5,8,8-tétraméthyl-2-naphtyl)-1-propynyl]-4-thiophenecarboxylique.
- Acide 2-[3-(3,5,5,8,8-pentaméthyl-5,6,7,8-tétrahydro-2-naphtyl)-1-propynyl]-4-thiophenecarboxylique.
- Acide 2-hydroxy-4-[3-(3-*tert*-butyl-4-methoxyphenyl)-1-propynyl]benzoïque
- Acide 2-hydroxy-4-[3-(3-*tert*-butyl-4-hydroxyphenyl)-1-propynyl]benzoïque.

Parmi les composés de formule (II) ci-dessus rentrant dans le cadre de la présente invention, on peut notamment citer les composés suivants:
- Acide 4-[3-(3,5-di-tert-butyl-4-oxo-2,5-cyclohexadiene-1-ylidene)-1-propynyl]benzoïque.
- Acide 2-hydroxy-4-[3-(3,5-di-tert-butyl-4-oxo-2,5-cyclohexadiene-1-ylidene)-1-propynyl]benzoïque.

Selon la présente invention les composés de formule (I) ou (II) plus particulièrement préférés sont ceux pour lesquels au moins l'une des, et de préférence toutes les, conditions suivantes est remplie :
- R₁ représente le radical -CO-R₇,
- Ar représente les radicaux de formule (a) ou (e).

Plus particulièrement, les composés de formule (I) préférés sont ceux pour lesquels R₈ et R₉, pris séparément, soit représentent des atomes d'hydrogène, soit l'un représente un atome d'hydrogène et l'autre un radical alkyle inférieur. De manière encore plus préférée, R₈ et R₉ représentent des atomes d'hydrogène.

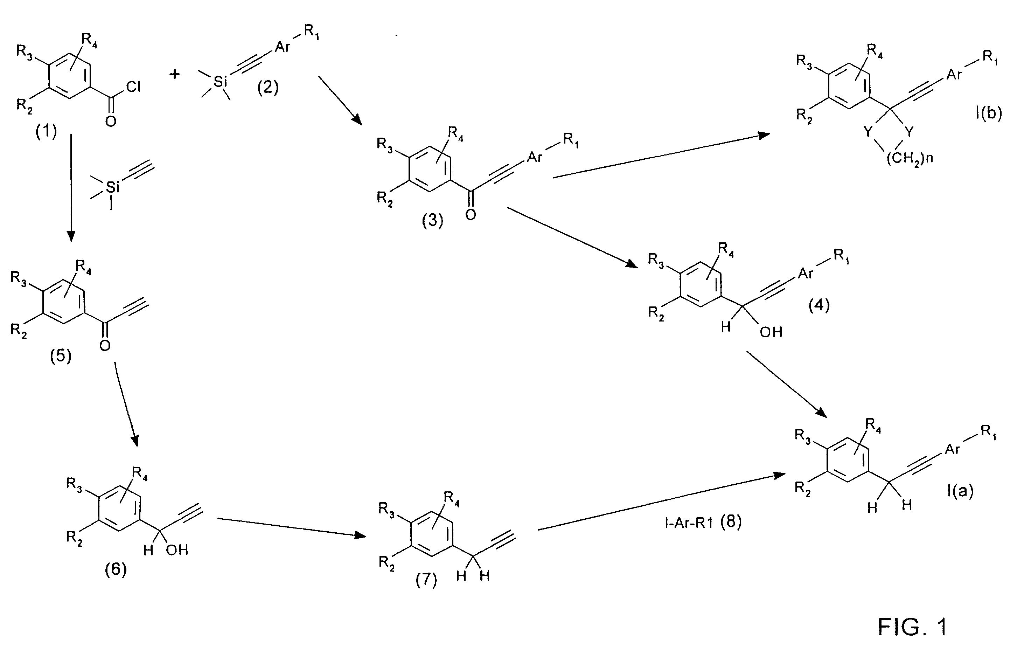

La présente invention a également pour objet les procédés de préparation des composés de formules (I) et (II) ci-dessus selon les schémas réactionnels donnés aux figures 1, 2 et 3.

Ainis, les dérivés de formule (la) peuvent être préparés (FIG. 1) par une suite de réactions comprenant l'action d'un chlorure de benzoyle de formule (1) avec un dérivé acétylénique de formule (2) en présence d'un acide de Lewis (par exemple AlCl₃) dans un solvant chloré, tel le dichlorométhane. La cétone (3) ainsi obtenue est réduite en alcool (4) par action d'un hydrure alcalin, tel le borohydrure de sodium, dans un solvant alcoolique (par exemple le méthanol). La réduction de la fonction alcool en carbure peut s'effectuer en présence d'iodure de triméthylsilane dans un solvant tel l'hexane ou par transfert d'hydrure à partir d'un silane, tel le triéthylsilane en présence de BF₃,Et₂O dans un solvant chloré tel le chlorure de méthylène.

Les dérivés de formule (la) peuvent être aussi préparés (FIG 1) par une suite de réactions comprenant l'action d'un chlorure de benzoyle de formule (1) avec le triméthylacétylénure de lithium en présence d'un acide de Lewis (par exemple AlCl₃) dans un solvant chloré, tel le dichlorométhane. La cétone (5) ainsi obtenue est d'abord réduite en alcool (6) par action d'un hydrure alcalin, tel le borohydrure de sodium, dans un solvant alcoolique (par exemple le méthanol) puis en carbure (7) par exemple par transfert d'hydrure à partir d'un silane, tel le triéthylsilane en présence de BF₃,Et₂O dans un solvant chloré tel le chlorure de méthylène. Puis, on procède au couplage du composé (7) avec un dérivé halogéné (8), de préférence iodé ou bromé en présence d'un catalyseur au Palladium [par exemple le chlorure de Bis-(triphénylphosphine)-palladium(II)] dans un solvant, tel la triéthylamine.

Les composés de formule (Ib) peuvent être obtenus (FIG 1) à partir du dérivé cétonique (3) par réaction avec un glycol (éthylèneglycol, propylèneglycol) ou un dithiol (éthanedithiol, propanedithiol) en présence de pyridinium para-toluènesulfonate dans un solvant aromatique tel le toluène avec entraînement azéotropique de l'eau formée.

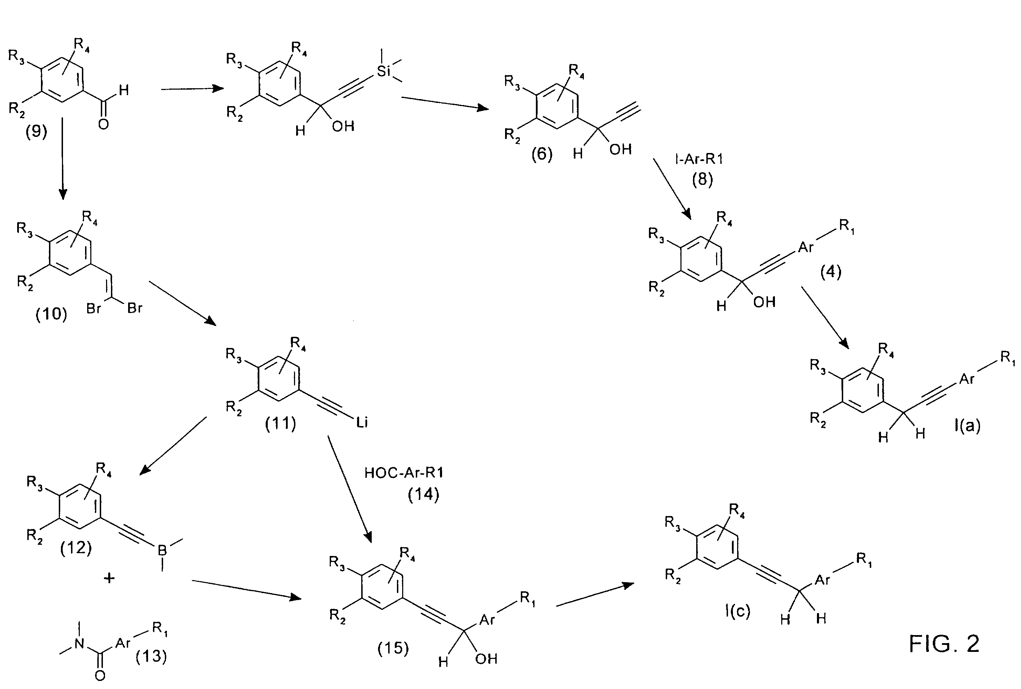

Les composés de formule (la) peuvent être encore préparés (FIG 2) par une suite de réactions comprenant l'action du triméthylsilyl acétylénure de lithuim sur les composés aldéhydiques (9) et déprotection avec du fluorure de tétrabutylammonium dans le THF et obtention de l'alcool propargylique (6). Puis, on procède au couplage avec un dérivé halogéné (8), de préférence iodé ou bromé en présence d'un catalyseur au Palladium [par exemple le chlorure de Bis-(triphénylphosphine)-palladium(II)] dans un solvant, tel la triéthylamine, et réduction de la fonction alcool en carbure comme précédemment.

Les composés de formule (Ic) peuvent être préparés (FIG 2) à partir des dérivés alcoolpropargyliques (15) par réduction de la fonction alcool en carbure comme précèdemment. Les dérivés alcoolpropargyliques (15) étant préparés:
- soit par action d'un acétylénure de bore (12) (préparé in situ à partir de phényl acétylénure de lithuim (11) et de trifluorure de bore à -78°C dans le THF) avec un benzamide tertiaire de formule (13) dans un solvant organique, tel le THF,
- soit par action du phényl acétylénure de lithium (11) sur les dérivés aldéhydiques (14).

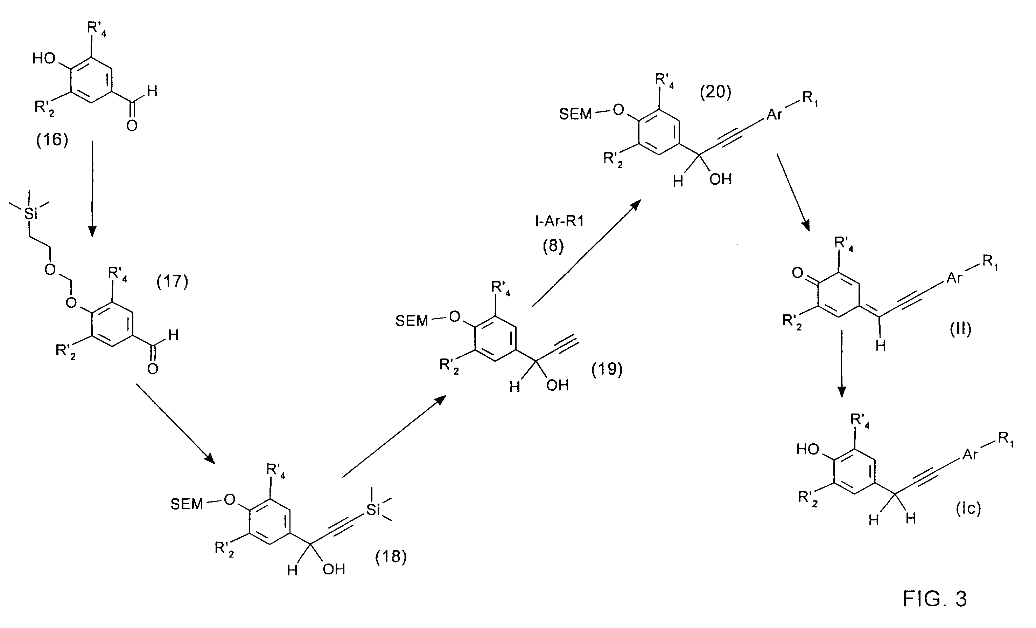

Les composés de formule (Ic) peuvent être préparés (FIG 3) à partir des composés de formule (II) par action d'un hydrure alcalin, tel le borohydrure de sodium, dans un solvant alcoolique (par exemple le méthanol). Les composés de formule (II) étant préparés par une suite de réactions à partir des composés hydroxybenzaldéhydes (16) comprenant la protection de la fonction phénol par action du chlorure de 2-(triméthylsilyl)éthoxyméthane (SEMCI) (17), puis l'action du triméthylsilyl acétylénure de lithuim (18) et la déprotection sélective du groupement triméthylsilyl porté par l'acétylénique avec du fluorure de tétrabutylammonium dans le THF et obtention de l'alcool propargylique (19). Par couplage avec un dérivé halogéné (8), de préférence iodé ou bromé en présence d'un catalyseur au Palladium [par exemple le chlorure de Bis-(triphénylphosphine)-palladium(II)] dans un solvant, tel la triéthylamine, on obtient le composé (20). La coupure du groupement protecteur (SEM) est effectuée avec de l'acide trifluoroacétique dans un solvant chloré, tel que le chlorure de méthylène

La présente invention a également pour objet à titre de médicament les composés de formules (I) et (II) tels que définis ci-dessus.

Ces composés présentent une activité dans le test de différenciation des cellules (F9) de tératocarcinome embryonnaire de souris (Cancer Research 43, p. 5268, 1983) et/ou dans le test d'inhibition de l'ornithine décarboxylase après induction par le TPA chez la souris (Cancer Research 38, p. 793-801, 1978). Ces tests montrent les activités des composés respectivement dans les domaines de la différenciation et de la prolifération cellulaire.

Ces composés présentent également des paramètres cinétiques très avantageux pour le domaine pharmaceutique par rapport à d'autres composés de synthèse de type rétinoïdes (le temps de demi-vie d'élimination et le temps de séjour moyen de ces composés dans l'organisme sont faibles)

Les composés de formule (I) ou (II) selon l'invention conviennent particulièrement bien dans les domaines de traitement suivants :
1) pour traiter les affections dermatologiques liées à un désordre de la kératinisation portant sur la différenciation et sur la prolifération notamment pour traiter les acnées vulgaires, comédoniennes, polymorphes, rosacées, les acnées nodulokystiques, conglobata, les acnées séniles, les acnées secondaires telles que l'acnée solaire, médicamenteuse ou professionnelle,
2) pour traiter d'autres types de troubles de la kératinisation, notamment les ichtyoses, les états ichtyosiformes, la maladie de Darrier, les kératodermies palmoplantaires, les leucoplasies et les états leucoplasiformes, le lichen cutané ou muqueux (buccal),
3) pour traiter d'autres affections dermatologiques liées à un trouble de la kératinisation avec une composante inflammatoire et/ou immuno-allergique et notamment toutes les formes de psoriasis qu'il soit cutané, muqueux ou unguéal, et même le rhumatisme psoriatique, ou encore l'atopie cutanée, telle que l'eczéma ou l'atopie respiratoire ou encore l'hypertrophie gingivale ; les composés peuvent également être utilisés dans certaines affections inflammatoires ne présentant pas de trouble de la kératinisation,
4) pour traiter toutes les proliférations dermiques ou épidermiques qu'elles soient bénignes ou malignes, qu'elles soient ou non d'origine virale telles que verrues vulgaires, les verrues planes et l'épidermodysplasie verruciforme, les papillomatoses orales ou florides et les proliférations pouvant être induites par les ultra-violets notamment dans le cas des épithélioma baso et spinocellulaires,
5) pour traiter d'autres désordres dermatologiques tels que les dermatoses bulleuses et les maladies du collagène,
6) pour traiter certains troubles ophtalmologiques, notamment les cornéopathies,
7) pour réparer ou lutter contre le vieillissement de la peau, qu'il soit photo-induit ou chronologique ou pour réduire les pigmentations et les kératoses actiniques, ou toutes pathologies associées au vieillissement chronologique ou actinique,
8) pour prévenir ou guérir les stigmates de l'atrophie épidermique et/ou dermique induite par les corticostéroïdes locaux ou systémiques, ou toute autre forme d'atrophie cutanée,
9) pour prévenir ou traiter les troubles de la cicatrisation ou pour prévenir ou pour réparer les vergetures,
10) pour lutter contre les troubles de la fonction sébacée tels que l'hyperséborrhée de l'acnée ou la séborrhée simple,
11) dans le traitement ou la prévention des états cancéreux ou précancéreux,
12) dans le traitement d'affections inflammatoires telles que l'arthrite,
13) dans le traitement de toute affection d'origine virale au niveau cutané, telle que le syndrome de Kaposis, ou général,
14) dans la prévention ou le traitement de l'alopécie,
15) dans le traitement d'affections dermatologiques ou générales à composante immunologique,
16) dans le traitement d'affections du système cardiovasculaire telles que l'artériosclérose ou l'hypertension ainsi que le diabète non-insulino dépendant,.
17) dans le traitement de désordres cutanés dus à une exposition aux rayonnements U.V..

Dans les domaines thérapeutiques mentionnés ci-dessus, les composés selon l'invention peuvent être avantageusement employés en combinaison avec d'autres composés à activité de type rétinoïde, avec les vitamines D ou leurs dérivés, avec des corticostéroïdes, avec des anti-radicaux libres, des α-hydroxy ou α-céto acides ou leurs dérivés, ou bien encore avec des bloqueurs de canaux ioniques. Par vitamines D ou leurs dérivés, on entend par exemple les dérivés de la vitamine D₂ ou D₃ et en particulier la 1,25-dihydroxyvitamine D₃. Par anti-radicaux libres, on entend par exemple l'α-tocophérol, la Super Oxyde Dismutate, l'Ubiquinol ou certains chélatants de métaux. Par α-hydroxy ou α-céto acides ou leurs dérivés, on entend par exemple les acides lactique, malique, citrique, glycolique, mandélique, tartrique, glycérique ou ascorbique ou leurs sels, amides ou esters. Enfin, par bloqueurs de canaux ioniques, on entend par exemple le Minoxidil (2,4-diamino-6-pipéridino-pyrimidine-3-oxyde) et ses dérivés.

La présente invention a également pour objet des compositions médicamenteuses contenant au moins un composé de formule (I) ou (II) telle que définie ci-dessus, l'un de ses isomères optiques ou géométriques ou un de ses sels.

La présente invention a donc ainsi pour objet une nouvelle composition médicamenteuse destinée notamment au traitement des affections susmentionnées, et qui est caractérisée par le fait qu'elle comprend, dans un support pharmaceutiquement acceptable et compatible avec le mode d'administration retenu pour cette dernière, au moins un composé de formule (I) ou (II), l'un de ses isomères optiques ou géométriques ou un de ses sels.

L'administration des composés selon l'invention peut être effectuée par voie entérale, parentérale, topique ou oculaire.

Par voie entérale, les médicaments peuvent se présenter sous forme de comprimés, de gélules, de dragées, de sirops, de suspensions, de solutions, de poudres, de granulés, d'émulsions, de microsphères ou de nanosphères ou de vésicules lipidiques ou polymériques permettant une libération contrôlée. Par voie parentérale, les compositions peuvent se présenter sous forme de solutions ou de suspensions pour perfusion ou pour injection.

Les composés selon l'invention sont généralement administrés à une dose journalière d'environ 0,01 mg/kg à 100 mg/Kg en poids corporel, et ceci à raison de 1 à 3 prises.

Par voie topique, les compositions pharmaceutiques à base de composés selon l'invention sont plus particulièrement destinées au traitement de la peau et des muqueuses et peuvent alors se présenter sous forme d'onguents, de crêmes, de laits, de pommades, de poudres, de tampons imbibés, de solutions, de gels, de sprays, de lotions ou de suspensions. Elles peuvent également se présenter sous forme de microsphères ou nanosphères ou vésicules lipidiques ou polymériques ou de patches polymériques et d'hydrogels permettant une libération contrôlée. Ces compositions par voie topique peuvent par ailleurs se présenter soit sous forme anhydre, soit sous une forme aqueuse, selon l'indication clinique.

Par voie oculaire, ce sont principalement des collyres.

Ces compositions à usage topique ou oculaire contiennent au moins un composé de formule (I) ou (II) telle que définie ci-dessus, ou l'un de ses isomères optiques ou géométriques ou encore l'un de ses sels, à une concentration de préférence comprise entre 0,001% et 5% en poids par rapport au poids total de la composition.

Les composés de formule (I) ou (II) selon l'invention trouvent également une application dans le domaine cosmétique, en particulier dans l'hygiène corporelle et capillaire et notamment pour le traitement des peaux à tendance acnéique, pour la repousse des cheveux, l'anti-chute, pour lutter contre l'aspect gras de la peau ou des cheveux, dans la protection contre les aspects néfastes du soleil ou dans le traitement des peaux physiologiquement sèches, pour prévenir et/ou pour lutter contre le vieillissement photo-induit ou chronologique.

Dans le domaine cosmétique, les composés selon l'invention peuvent par ailleurs être avantageusement employés en combinaison avec d'autres composés à activité de type rétinoïde, avec les vitamines D ou leurs dérivés, avec des corticostéroïdes, avec des anti-radicaux libres, des α-hydroxy ou α-céto acides ou leurs dérivés, ou bien encore avec des bloqueurs de canaux ioniques, tous ces différents produits étant tels que définis ci-avant.

La présente invention vise donc également une composition cosmétique qui est caractérisée par le fait qu'elle comprend, dans un support cosmétiquement acceptable et convenant à une application topique, au moins un composé de formule (I) ou (II) telle que définie ci-dessus ou l'un de ses isomères optiques ou géométriques ou l'un de ses sels, cette composition cosmétique pouvant notamment se présenter sous la forme d'une crème, d'un lait, d'une lotion, d'un gel, de microsphères ou nanosphères ou vésicules lipidiques ou polymériques, d'un savon ou d'un shampooing.

La concentration en composé de formule (I) ou (II) dans les compositions cosmétiques selon l'invention est avantageusement comprise entre 0,001% et 3% en poids par rapport à l'ensemble de la composition.

Les compositions médicamenteuses et cosmétiques selon l'invention peuvent en outre contenir des additifs inertes ou même pharmacodynamiquement ou cosmétiquement actifs ou des combinaisons de ces additifs, et notamment : des agents mouillants; des agents dépigmentants tels que l'hydroquinone, l'acide azélaïque, l'acide caféïque ou l'acide kojique; des émollients; des agents hydratants comme le glycérol, le PEG 400, la thiamorpholinone, et ses dérivés ou bien encore l'urée; des agents antiséborrhéiques ou antiacnéiques, tels que la S-carboxyméthylcystéine, la S-benzyl-cystéamine, leurs sels ou leurs dérivés, ou le péroxyde de benzoyle; des antibiotiques comme l'érythromycine et ses esters, la néomycine, la clindamycine et ses esters, les tétracyclines; des agents antifongiques tels que le kétokonazole ou les polyméthylène-4,5 isothiazolidones-3; des agents favorisant la repousse des cheveux, comme le Minoxidil (2,4-diamino-6-pipéridino-pyrimidine-3-oxyde) et ses dérivés, le Diazoxide (7-chloro 3-méthyl 1,2,4-benzothiadiazine 1,1-dioxyde) et le Phénytoïn (5,4-diphénylimidazolidine 2,4-dione); des agents anti-inflammatoires non stéroïdiens; des caroténoïdes et, notamment, le β-carotène; des agents anti-psoriatiques tels que l'anthraline et ses dérivés; et enfin les acides eicosa-5,8,11,14-tétraynoïque et eicosa-5,8,11-trynoïque, leurs esters et amides.

Les compositions selon l'invention peuvent également contenir des agents d'amélioration de la saveur, des agents conservateurs tels que les esters de l'acide parahydroxybenzoïque, les agents stabilisants, des agents régulateurs d'humidité, des agents régulateurs de pH, des agents modificateurs de pression osmotique, des agents émulsionnants, des filtres UV-A et UV-B, des antioxydants, tels que l'a-tocophérol, le butylhydroxyanisole ou le butylhydroxytoluène.

On va maintenant donner, à titre d'illustration et sans aucun caractère limitatif, plusieurs exemples d'obtention de composés actifs de formule (I) ou (II) selon l'invention, ainsi que diverses formulations concrètes à base de tels composés. Dans ce qui suit ou ce qui précède, les pourcentages sont donnés en poids, sauf mention contraire.

### EXEMPLE 1

### Acide 4-[3-(3,5-di-tert-butyl-4-oxo-2,5-cyclohexadiene-1-ylidene)--1-propynyl]benzoïque.

### (a) 3,5-di-tert-butyl-4-(2-triméthylsilyléthoxyméthoxy)benzaldéhyde.

Dans un ballon, on introduit 12,3 g (52 mmoles) de 3,5-di-tert-butyl-4-hydroxybenzaldéhyde et 100 ml de THF. On ajoute sucessivement 10 ml (58 mmoles) de diisopropyléthylamine et 10 ,3 ml (58 mmoles) de chlorure de 2-triméthylsilylethoxyméthane et on chauffe à reflux pendant trois heures. On verse le milieu réactionnel dans l'eau, on extrait avec de l'éther éthylique, on décante la phase organique, on sèche sur sulfate de magnésium et on évapore. Le résidu obtenu est purifié par chromatographie sur colonne de silice élué avec un mélange d'acétate d'éthyle et d'hexane (3/97% en volume). Après évaporation des solvants, on recueille 15,6 g (82%) du produit attendu sous forme d'une huile incolore.

### (b) α-Triméthylsilyléthynyl-3,5-di-tert-butyl-4-(2-triméthylsilyléthoxyméthoxy) benzèneméthanol.

Dans un tricol, on introduit 6,6 ml (46,5 mmoles) de triméthylsilylacétylène et 50 ml de THF. A -78°C et sous courant d'azote, on ajoute goutte à goutte une solution de 18,6 ml (46,5 mmoles) de n-butyllithium (2,5 M dans l'hexane) et on laisse revenir à température ambiante.
Cette solution est introduite goutte à goutte dans une solution de 15,4 g (42,3 mmoles) de 3,5-di-tert-butyl-4-(2-triméthylsilyléthoxyméthoxy)benzaldéhyde dans 50 ml de THF à -78°C. On laisse le milieu réactionnel revenir à température ambiante, on le verse dans une solution aqueuse de chlorure d'ammonium, extrait avec de l'éther éthylique, on décante la phase organique, on sèche sur sulfate de magnésium, et on évapore. On obtient 18,5 g (95%) de l'alcool attendu sous forme d'une huile jaune.

### (c) α-éthynyl-3,5-di-tert-butyl-4-(2-triméthylsilyléthoxyméthoxy)benzèneméthanol

Dans un ballon, on introduit 18,5 g (40 mmoles) d'α-Triméthylsilyléthynyl-3,5-di-tert-butyl-4-(2-triméthylsilyléthoxyméthoxy) benzèneméthanol 50 ml de THF et on ajoute goutte à goutte 40 ml (44 mmoles) d'une solution de fluorure de tétrabutylammonium (1,1 M dans le THF). On agite à température ambiante une heure, on verse le milieu réactionnel dans l'eau, on extrait avec de l'éther éthylique, on décante la phase organique, et on sèche sur sulfate de magnésium, évapore. Le résidu obtenu est purifié par chromatographie sur colonne de silice élué avec un mélange d'acétate d'éthyle et d'hexane (5-95 en volume). Après évaporation des solvants, on recueille 13,5 g (86%) d'α-éthynyl-3,5-di-tert-butyl-4-(2-triméthylsilyléthoxyméthoxy)benzène méthanol sous forme d'une huile incolore.

### (d) 4-[[3-hydroxy-3-[3,5-di-tert-butyl-4-(2-triméthylsilyléthoxyméthoxy)phényl]-1-propynyl]]benzoate de méthyle.

Dans un tricol, on introduit 6 g (15,4 mmoles) d'α-éthynyl-3,5-di-tert-butyl-4-(2-triméthylsilyléthoxyméthoxy)benzèneméthanol, 4,1 g (15,4 mmoles) de 4-iodobenzoate de méthyle et 50 ml de triéthylamine. On dégaze le milieu réactionnel avec de l'azote pendant 30 minutes, puis on ajoute successivement 820 mg (1,2 mmole) de Bis(triphénylphosphine)palladium(II)chlorure et 360 mg (1,9 mmole) d'iodure de cuivre. On agite à température ambiante pendant quatre heures, on évapore à sec le milieu réactionnel, on reprend le résidu obtenu par l'eau et l'éther éthylique. On décante la phase organique, on sèche sur sulfate de magnésium, puis on évapore. Le résidu obtenu est purifié par chromatographie sur colonne de silice élué avec un mélange de dichlorométhane et d'heptane (80/20% en volume), on recueille 6,7 g (84%) de 4-[[3-hydroxy-3-[3,5-di-tert-butyl-4-(2-triméthylsilyléthoxyméthoxy)phényl]-1-propynyl]]benzoate de méthyle de point de fusion 91-2°C.

### (e) acide 4-[[3-hydroxy-3-[3,5-di-tert-butyl-4-(2-triméthylsilyléthoxyméthoxy)-phényl]-1-propynyl]]benzoïque.

Dans un ballon, on introduit 2,4 g (4,6 mmoles) de l'ester précédent 8,4 g (200 mmoles) d'hydroxyde de lithium et 100 ml de THF. On chauffe à reflux pendant 18 heures et on évapore à sec le milieu réactionnel. On reprend le résidu par l'eau, on l'acidifie à pH 1, on extrait avec de l'éther éthylique, on décante la phase organique, on sèche sur sulfate de magnésium, et on évapore. On triture le résidu dans l'heptane, filtre et recueille 2,2 g (94%) de l'acide attendu de point de fusion 155-6°C.

### (f) acide 4-[3-(3,5-di-tert-butyl-4-oxo-2,5-cyclohexadiene-1-ylidene)-1-propynyl] benzoïque.

Dans un tricol, on introduit 2,2 g (4,7 mmoles) d'acide 4-[[3-hydroxy-3-[3,5-di-tert-butyl-4-(2-triméthylsilyléthoxyméthoxy)phényl]-1-propynyl]]benzoïque et 75 ml de dichlorométhane. On ajoute à -78°C, 360 µl (4,7 mmoles) d'acide trifluoroacétique et on laisse remonter à température ambiante. On verse le milieu réactionnel dans l'eau, on extrait avec de l'éther éthylique, on décante la phase organique, on sèche sur sulfate de magnésium, et on évapore. Le résidu obtenu est trituré dans l'heptane, filtré, puis séché. On recueille 1,6 g (90%) d'acide 4-[3-(3,5-di-tert-butyl-4-oxo-2,5-cyclohexadiene-1-ylidene)-1-propynyl] benzoïque de point de fusion 216-8°C.

### EXEMPLE 2

### Acide 4-[3-(3,5-di-tert-butyl-4-hydroxyphenyl)-1-propynyl]benzoïque.

Dans un tricol, on introduit 756 mg (2 mmoles) d'acide 4-[3-(3,5-di-tert-butyl-4-oxo-2,5-cyclohexadiene-1-ylidene)-1-propynyl] benzoïque et 50 ml d'un mélange (50-50) de THF et de méthanol. A 0°C, on ajoute 152 mg (4 mmoles) de borohydrure de sodium et on laisse remonter à température ambiante. On verse le milieu réactionnel dans l'eau, on extrait avec de l'éther éthylique, on décante la phase organique, on sèche sur sulfate de magnésium, et on évapore. Le résidu obtenu est trituré dans l'heptane à reflux, filtré et séché. On recueille 510 mg (64%) d'acide 4-[3-(3,5-di-tert-butyl-4-hydroxy phenyl)-1-propynyl] benzoïque de point de fusion 198-9°C.

### EXEMPLE 3

### Acide 2-hydroxy-4-[3-(3,5-di-tert-butyl-4-oxo-2,5-cyclohexadiene-1-ylidene)-1-propynyl]benzoïque.

### (a) 2-hydroxy-4-[[3-hydroxy-3-[3,5-di-tert-butyl-4-(2-triméthylsilyléthoxyméthoxy) phényl]-1-propynyl]]benzoate de méthyle.

De manière analogue à l'exemple 1(d) par réaction de 6,7 g (17,3 mmoles) d'a-éthynyl-3,5-di-tert-butyl-4-(2-triméthylsilyléthoxyméthoxy)benzèneméthanol avec 4,8 g (17,3 mmoles) de 2-hydroxy-4-iodobenzoate de méthyle, on obtient 8,5 g (91%) de l'ester attendu sous forme d'une hulie jaune.

### (b) acide 2-hydroxy-4-[[3-hydroxy-3-[3,5-di-tert-butyl-4-(2-triméthylsilyléthoxy méthoxy)phényl]-1-propynyl]]benzoïque.

De manière analogue à l'exemple 1(e) à partir de 8,4 g (15,5 mmoles) de 2-hydroxy-4-[[3-hydroxy-3-[3,5-di-tert-butyl-4-(2-triméthylsilyléthoxyméthoxy) phényl]-1-propynyl]]benzoate de méthyle, on obtient 7,4 g (91%) de l'acide attendu de point de fusion 146-7°C.

### (c) acide 2-hydroxy-4-[3-(3,5-di-tert-butyl-4-oxo-2,5-cyclohexadiene-1-ylidene)-1-propynyl]benzoïque.

De manière analogue à l'exemple 1(f) à partir de 2,6 g (5 mmoles) d'acide 2-hydroxy-4-[[3-hydroxy-3-[3,5-di-tert-butyl-4-(2-triméthylsilyléthoxyméthoxy)phényl]-1-propynyl]]benzoïque, on obtient 1,7 g (89%) d'acide 2-hydroxy-4-[3-(3,5-di-tert-butyl-4-oxo-2,5-cyclohexadiene-1-ylidene)-1-propynyl]benzoïque de point de fusion 203°C et décomposition.

### EXEMPLE 4

### Acide 2-hydroxy-4-[3-(3,5-di-tert-butyl-4-hydroxyphenyl)-1-propynyl]benzoique.

De manière analogue à l'exemple 2 à partir de 1 g (2,6 mmoles d'acide 2-hydroxy-4-[3-(3,5-di-tert-butyl-4-oxo-2,5-cyclohexadiene-1-ylidene)-1-propynyl] benzoïque, on obtient 510 mg (51%) d'acide 2-hydroxy-4-[3-(3,5-di-tert-butyl-4-hydroxyphenyl)-1-propynyl] benzoïque de point de fusion 205-6°C.

### EXEMPLE 5

### Acide 4-[3-(5,6,7,8-tétrahydro-5,5,8,8-tétraméthyl-2-naphtyl)-1-propynyl] benzoïque.

### (a) 4-triméthylsilyléthynylbenzoate de méthyle.

Dans un tricol et sous courant d'azote, on introduit 21,5 g (0,1 mole) de 4-bromobenzoate de méthyle, 300 ml de triéthylamine et un mélange de 200 mg d'acétate de palladium et de 400 mg de triphénylphosphine. On ajoute ensuite 20 g (0,20 mole) de triméthylsilylacétylène, on chauffe progressivement à 90°C durant 1 heure et on laisse à cette température pendant 5 heures. On refroidit le milieu réactionnel, on filtre le sel et on évapore. On reprend le résidu avec 200 ml d'acide chlorhydrique (5%) et 400 ml d'éther éthylique. On décante la phase éthérée, on lave à l'eau, on sèche sur sulfate de magnésium, et on évapore. Le résidu obtenu est purifié par chromatographie sur colonne de silice, élué avec du dichlorométhane. Après évaporation des solvants, on recueille 23 g (100%) du dérivé attendu sous forme d'une huile incolore.

### (b) 4-[3-oxo-3-(5,6,7,8-tétrahydro-5,5,8,8-tétraméthyl-2-naphtyl)-1-propynyl] benzoate de méthyle.

Dans un ballon, on introduit 8,4 g (36 mmoles) de chlorure de 5,6,7,8-tétrahydro-5,5,8,8-tétraméthyl-2-naphtoyle, 6,9 g (29,7 mmoles) de 4-triméthylsilyléthynylbenzoate de méthyle et 100 ml de dichlorométhane. On ajoute à 0°C, par petites quantités, 16,8 g (125 mmoles) d'AlCl₃ et on agite à température ambiante pendant 8 heures. On verse le milieu réactionnel dans la glace, on extrait avec du dichlorométhane, on décante la phase organique, on sèche sur sulfate de magnésium, et on évapore. Le résidu obtenu est purifié par chromatographie sur colonne de silice élué avec un mélange de dichlorométhane et d'hexane (50/50 % en volume). On recueille 6,8 g (61%) de produit attendu, de point de fusion 113-4°C.

### (c) 4-[3-hydroxy-3-(5,6,7,8-tétrahydro-5,5,8,8-tétraméthyl-2-naphtyl)-1-propynyl] benzoate de méthyle.

Dans un ballon, on introduit 4,7 g (125 mmoles) du produit obtenu précedemment et 100 ml de méthanol. Tout en refroidissant à 0°C, on ajoute successivement 5,7 g (150 mmoles) de CeCl₃,7H₂O et 530 mg (125 mmoles) de borohydrure de sodium et on agite à température ambiante pendant 4 heures. On verse le milieu réactionnel dans un mélange eau-éther éthylique, décante la phase organique, on lave à l'eau, on sèche sur sulfate de magnésium, et on évapore. Le résidu obtenu est trituré dans 100 ml d'hexane, filtré et séché. On recueille 4 g (85%) du produit attendu de point de fusion 142-3°C.

### (d) acide 4-[3-hydroxy-3-(5,6,7,8-tétrahydro-5,5,8,8-tétraméthyl-2-naphtyl)-1-propynyl]benzoïque.

De manière analogue à l'exemple 1(e) à partir de 1,7 g (4,5 mmoles) de l'ester méthylique précédent, on obtient 1,3 g (79%) d'acide 4-[3-hydroxy-3-(5,6,7,8-tétrahydro-5,5,8,8-tétraméthyl-2-naphtyl)-1-propynyl]benzoïque de point de fusion 146-7°C.

### (e) acide 4-[3-(5,6,7,8-tétrahydro-5,5,8,8-tétraméthyl-2-naphtyl)-1-propynyl] benzoïque.

Dans un tricol et sous courant d'azote, on introduit 2,1 ml (8,1 mmoles) de BF₃-Et₂O (48%) et 50 ml de dichlorométhane. A -20°C, on ajoute 2,6 ml (16,2 mmoles) de triéthylsilane puis une solution de 1g (2,7 mmoles) d'acide 4-[3-hydroxy-3-(5,6,7,8-tétrahydro-5,5,8,8-tétraméthyl-2-naphtyl)-1-propynyl] benzoïque dans 30 ml de dichlorométhane et on agite à température ambiante pendant 30 minutes. On verse le milieu réactionnel dans l'eau, on extrait avec de l'éther éthylique, on décante la phase organique, on sèche sur sulfate de magnésium, et on évapore. Le résidu obtenu est purifié sur colonne de silice élué avec du dichlorométhane. Après évaporation des solvants, on recueille 780 mg (82%) d'acide 4-[3-(5,6,7,8-tétrahydro-5,5,8,8-tétraméthyl-2-naphtyl)-1-propynyl] benzoïque de point de fusion 167-8°C.

### EXEMPLE 6

### 2-hydroxy-4-[3-(5,6,7,8-tétrahydro-5,5,8,8-tétraméthyl-2-naphtyl)-1-propynyl] benzoate de méthyle.

### (a) α-Triméthylsilyléthynyl-(5,6,7,8-tétrahydro-5,5,8,8-tétraméthyl-2-naphtalène) méthanol.

Dans un tricol, on introduit 17,13 ml (0,121 mole) de triméthylsilylacétylène et 100 ml de THF. A -78°C et sous courant d'azote, on ajoute goutte à goutte une solution de 48,5 ml (0,121 mole) de n-butyllithium (2,5 M dans l'hexane) et on laisse revenir à température ambiante.
Cette solution est introduite goutte à goutte dans une solution de 23,8 g (0,11 mole) de 5,6,7,8-tétrahydro-5,5,8,8-tétraméthyl-2-naphtalènecarboxaldéhyde dans 100 ml de THF à -78°C. On laisse le milieu réactionnel revenir à température ambiante, on le verse dans une solution aqueuse de chlorure d'ammonium, on extrait avec de l'éther éthylique, on décante la phase organique, on sèche sur sulfate de magnésium, et on évapore. Le résidu obtenu est purifié par chromatographie sur colonne de silice élué avec un mélange de dichlorométhane et d'hexane (50/50% en volume). Après évaporation des solvants, on recueille 29,9 g (86%) de l'alcool attendu sous forme d'une huile jaune.

### (b) α-éthynyl-(5,6,7,8-tétrahydro-5,5,8,8-tétraméthyl-2-naphtalène)méthanol.

Dans un ballon, on introduit 29,9 g (95,2 mmoles) d'a-Triméthylsilyléthynyl-(5,6,7,8-tétrahydro-5,5,8,8-tétraméthyl-2-naphtalène)méthanol 100 ml de THF et ajoute goutte à goutte 103,8 ml (114,2 mmoles) d'une solution de fluorure de tétrabutylammonium (1,1 M dans le THF). On agite à température ambiante une heure, on verse le milieu réactionnel dans l'eau, on extrait avec de l'éther éthylique, on décante la phase organique, on sèche sur sulfate de magnésium, et on évapore. Le résidu obtenu est purifié par chromatographie sur colonne de silice élué avec un mélange d'acétate d'éthyle et d'hexane (1/4 en volume). Après évaporation des solvants, on recueille 18,1 g (79%) d'α-éthynyl-(5,6,7,8-tétrahydro-5,5,8,8-tétraméthyl-2-naphtalène) méthanol de point de fusion 56-7°C.

### (c) 2-hydroxy-4-[3-hydroxy-3-(5,6,7,8-tétrahydro-5,5,8,8-tétraméthyl-2-naphtyl)-1-propynyl] benzoate de méthyle.

De manière anlogue à l'exemple 1(d) par réaction de 10,3 g (42,5 mmoles) d'a-éthynyl-(5,6,7,8-tétrahydro-5,5,8,8-tétraméthyl-2-naphtalène)méthanol avec 11,8 g (42,5 mmoles) de 2-hydroxy-4-iodobenzoate de méthyle, on obtient 13,6 g (82%) de l'ester méthylique attendu de point de fusion 92-3°C.

### (d) 2-hydroxy-4-[3-(5,6,7,8-tétrahydro-5,5,8,8-tétraméthyl-2-naphtyl)-1-propynyl] benzoate de méthyle.

De manière analogue à l'exemple 5 (e) à partir de 1 g (2,6 mmoles) de l'ester méthylique précédent, on obtient 210 mg (22%) de 2-hydroxy-4-[3-(5,6,7,8-tétrahydro-5,5,8,8-tétraméthyl-2-naphtyl)-1-propynyl] benzoate de méthyle de point de fusion 75-7°C.

### EXEMPLE 7

### Acide 2-hydroxy-4-[3-(5,6,7,8-tétrahydro-5,5,8,8-tétraméthyl-2-naphtyl)--1-propynylbenzoïque.

### (a) acide 2-hydroxy-4-[3-hydroxy-3-(5,6,7,8-tétrahydro-5,5,8,8-tétraméthyl-2-naphtyl)-1-propynyl]benzoïque.

De manière à l'exemple 1(e) à partir de 8,5 g (21,6 mmoles) de 2-hydroxy-4-[3-hydroxy-3-(5,6,7,8-tétrahydro-5,5,8,8-tétraméthyl-2-naphtyl)-1-propynyl]benzoate de méthyle, on obtient 7,8 g (95%) de l'acide attendu de point de fusion 203° avec décomposition.

### (b) acide 2-hydroxy-4-[3-(5,6,7,8-tétrahydro-5,5,8,8-tétraméthyl-2-naphtyl)-1-propynyl]benzoïque.

De manière analogue à l'exemple 5 (e) à partir de 1 g (2,6 mmoles) d'acide 2-hydroxy-4-[3-hydroxy-3-(5,6,7,8-tétrahydro-5,5,8,8-tétraméthyl-2-naphtyl)-1-propynyl]benzoïque, on obtient 820 mg (86%) d'acide 2-hydroxy-4-[3-(5,6,7,8-tétrahydro-5,5,8,8-tétraméthyl-2-naphtyl)-1-propynyl]benzoïque de point de fusion 178-80°C.

### EXEMPLE 8

### Acide 2-hydroxy-4-[3-(3-hydroxy-5,6,7,8-tétrahydro-5,5,8,8-tétraméthyl-2-naphtyl)--1-propynyl/benzoique.

### (a) 3-méthoxyéthoxyméthoxy-5,6,7,8-tétrahydro-5,5,8,8-tétraméthyl-2-bromo-naphtalène.

Dans un tricol et sous courant d'azote, on introduit 720 mg (24 mmoles) d'hydrure de sodium (80% dans l'huile et 50 ml de DMF. On ajoute goutte à goutte une solution de 5,7 g (20 mmoles) de 3-bromo-5,6,7,8-tétrahydro-5,5,8,8-tétraméthyl-2-naphtol dissous dans 75 ml de DMF et on agite jusqu'à cessation du dégagement gazeux. A 0°C, on ajoute ensuite 6,8 ml (59,3 mmoles) de chlorure de méthoxyéthoxyméthane et on agite pendant quatre heures. On verse le milieu réactionnel dans l'eau, on extrait avec de l'éther éthylique, on décante la phase organique, on sèche sur sulfate de magnésium, et on évapore. On recueille 16,6 g (91%) du produit attendu sous forme d'une huile.

### (b) 3-méthoxyéthoxyméthoxy-5,6,7,8-tétrahydro-5,5,8,8-tétraméthyl-2-naphtyl carboxaldéhyde.

Dans un tricol et sous courant d'azote, on introduit 16,3 g (44 mmoles) de 3-méthoxyéthoxyméthoxy-5,6,7,8-tétrahydro-5,5,8,8-tétraméthyl-2-bromonaphtalène et 50 ml de THF. A - 78°C, on ajoute goutte à goutte 19,3 ml de n-butyllithium (2,5 M dans l'hexane) et on agite 30 minutes, puis on ajoute 3,7 ml (48,4 mmoles) de DMF et on laisse remonter à température ambiante. On verse le milieu réactionnel dans une solution aqueuse de chlorure d'ammonium, on extrait avec de l'éther éthylique, on décante la phase organique, on sèche sur sulfate de magnésium, et on évapore. On recueille 13,9 g (100%) de l'aldéhyde attendu sous forme d'une huile.

### c) α-Triméthylsilyléthynyl-(3-méthoxyéthoxyméthoxy-5,6,7,8-tétrahydro-5,5,8,8-tétraméthyl-2-naphtalène)méthanol.

De manière analogue à l'exemple 1(b) par réaction de 13,5 g (42,1 mmoles) de 3-méthoxyéthoxyméthoxy-5,6,7,8-tétrahydro-5,5,8,8-tétraméthyl-2-naphtylcarboxaldéhyde avec 7,1 ml (50,6 mmoles) de triméthylsilylacétylène, on obtient 17,5 g (100%) de l'alcool attendu sous forme d'une huile jaune.

### (d) α-éthynyl-(3-méthoxyéthoxyméthoxy-5,6,7,8-tétrahydro-5,5,8,8-tétraméthyl-2-naphtalène)méthanol.

De manière analogue à l'exemple 1(c) à partir de 17 g (40,6 mmoles) d'α-Triméthylsilyléthynyl-(3-méthoxyéthoxyméthoxy-5,6,7,8-tétrahydro-5,5,8,8-tétraméthyl-2-naphtalène)méthanol, on obtient 12,4 g (88%) de α-éthynyl-(3-méthoxyéthoxyméthoxy-5,6,7,8-tétrahydro-5,5,8,8-tétraméthyl-2-naphtalène) méthanol sous forme d'une huile.

### (e) 2-hydroxy-4-[3-hydroxy-3-(3-méthoxyéthoxyméthoxy-5,6,7,8-tétrahydro-5,5,8,8-tétraméthyl-2-naphtyl)-1-propynyl]benzoate de méthyle.

De manière analogue à l'exemple 1(d) par réaction de 7,1 g (20,5 mmoles) de α-éthynyl-(3-méthoxyéthoxyméthoxy-5,6,7,8-tétrahydro-5,5,8,8-tétraméthyl-2-naphtalène)méthanol avec 5,7 g (20,5 mmoles) de 2-hydroxy-4-iodobenzoate de méthyle, on obtient 9,6 g (94%) de l'ester méthylique sous forme d'une huile.

### (f) acide 2-hydroxy-4-[3-hydroxy-3-(3-méthoxyéthoxyméthoxy-5,6,7,8-tétrahydro-5,5,8,8-tétraméthyl-2-naphtyl)-1-propynyl]benzoïque.

De manière analogue à l'exemple 1 (e) à partir de 9 g (21,6 mmoles) de l'ester méthylique précédent, on obtient 7,8 g (89%) de l'acide attendu de point de fusion 106-8°C.

### (g) acide 2-hydroxy-4-[3-(3-hydroxy-5,6,7,8-tétrahydro-5,5,8,8-tétraméthyl-2-naphtyl)-1-propynyl]benzoïque.

De manière analogue à l'exemple 5(e) à partir de 1,7 g (3,5 mmoles) d'acide 2-hydroxy-4-[3-hydroxy-3-(3-méthoxyéthoxyméthoxy-5,6,7,8-tétrahydro-5,5,8,8-tétraméthyl-2-naphtyl)-1-propynyl]benzoïque, on obtient 670 mg (50%) de l'acide attendu de point de fusion 216-7°C.

### EXEMPLE 9

### 2-hydroxy-4-[3-(5,6,7,8-tétrahydro-5,5,8,8-tétraméthyl-2-naphtyl)-1-propynyl)benzèneméthanol.

Dans un tricol et sous courant d'azote, on introduit 760 mg (2,2 mmoles)de 2-hydroxy-4-[3-(5,6,7,8-tétrahydro-5,5,8,8-tétraméthyl-2-naphtyl)-1-propynyl] benzoate de méthyle et 20 ml de toluène. A -78°C, on ajoute 4,4 ml d'hydrure de diisobutylaluminium (1 M dans le toluène) et laisse remonter à température ambiante. On introduit sucessivement 9 ml de méthanol, puis 9 ml d'acide chlorhydrique (1N), On verse le milieu réactionnel dans un mélange acétate d'éthyle-eau, on décante la phase organique, on sèche sur sulfate de magnésium, puis on évapore. Le résidu est purifié par chromatographie sur colonne de silice élué avec un mélange d'acétate d'éthyle et d'heptane (50/50% en volume). Après évaporation des solvants, on recueille 200 mg (30%) d'alcool attendu de point de fusion 94-5°C.

### EXEMPLE 10

### 2-hydroxy-4-[3-(5,6,7,8-tétrahydro-5,5,8,8-tétraméthyl-2-naphtyl)-1-propynyl]benzoate de diéthanolamine.

Dans un ballon, on introduit 100 mg (2,76 mmoles) d'acide 2-hydroxy-4-[3-(5,6,7,8-tétrahydro-5,5,8,8-tétraméthyl-2-naphtyl)-1-propynyl]benzoïque 5 ml de méthanol et ajoute 29 mg (2,76 mmoles) de diéthanolamine. On agite pendant une heure, évapore à sec le milieu réactionnel, triture le résidu obtenu dans un mélange d'heptane et d'éther éthylique (50-50). On filtre le solide et le sèche. On recueille 100 mg (78%) de sel de diéthanolamine de point de fusion 100-5°C.

### EXEMPLE 11

### 2-hydroxy-4-[3-(5,6,7,8-tétrahydro-5,5,8,8-tétraméthyl-2-naphtyl)-1-propynyl)benzoate de lithium.

De manière analogue à l'exemple 10 par réaction de 200 mg (5,5 mmoles) d'acide 2-hydroxy-4-[3-(5,6,7,8-tétrahydro-5,5,8,8-tétraméthyl-2-naphtyl)-1-propynyl]benzoïque avec 23 mg (5,5 mmoles) d'hydroxyde de lithium hydraté, on obtient 150 mg (74%) de sel de lithium attendu de point de fusion 225-9°C.

### EXEMPLE 12

### Acide 4-[3-(5,6,7,8-tétrahydro-5,5,8,8-tétraméthyl-2-naphtyl-2-propynyl) benzoïque.

De manière analogue à l'exemple 5(e) à partir de 1,66 g (4,6 mmoles) d'acide 4-[1-hydroxy-3-(5,6,7,8-tétrahydro-5,5,8,8-tétraméthyl-2-naphtyl)-2-propynyl] benzoïque (préparé à l'exemple 10(b) du brevet EP 0 661 258), on obtient 310 mg (19,5%) d'acide 4-[3-(5,6,7,8-tétrahydro-5,5,8,8-tétraméthyl-2-naphtyl)-2-propynyl]benzoïque de point de fusion 159-60°C.

### EXEMPLE 13

### Acide 2-hydroxy-4-[3-(4,4-dimethylthiochromane-6-yl)-1-propynyl]benzoique

De manière analogue à l'exemple 5(e) à partir de 750 mg (2 mmoles) d'acide 2-hydroxy-4-[3-hydroxy-3-(4,4-diméthylthiochroman-6-yl)-1-propynyl]benzoïque (préparé à l'exemple19 du brevet EP 0 661 258), on obtient après chromatographie sur colonne de silice élué avec un mélange de dichlorométhane et de méthanol (80-20) 340 mg (28%) d'acide 2-hydroxy-4-[3-(4,4-dimethylthiochromane-6-yl)-1-propynyl]benzoïque de point de fusion 195-6°C.

### EXEMPLE 14

### Acide 2-hydroxy-4-[3-(8,8-diméthyl-5,6,7,8-tétrahydro-2-naphtyl)-1-propynyl]benzoïque.

### (a) 2-hydroxy-4-[3-oxo-3-(8,8-diméthyl-5,6,7,8-tétrahydro-2-naphtyl)-1-propynyl] benzoate de méthyle.

Dans un ballon, on introduit 12 g (54 mmoles) de chlorure de 8,8-diméthyl-5,6,7,8-tétrahydro-2-naphtoyle 14,7 g (59 mmoles) de 2-hydroxy-4-triméthylsilyléthynylbenzoate de méthyle (préparé à l'exemple 5(a) du brevet EP 0 661 258) et 200 ml de dichlorométhane. On ajoute à 0°C par petites quantités 21,6 g (162 mmoles) d'AlCl₃ et agite à température ambiante pendant 8 heures. On verse le milieu réactionnel dans la glace, extrait avec du dichlorométhane, décante la phase organique, sèche sur sulfate de magnésium, évapore. Le résidu obtenu est purifié par chromatographie sur colonne de silice élué avec un mélange de dichlorométhane et d'hexane (50-50). On recueille 12,5 g (64%) de produit attendu, de point de fusion 114-6°C.

### (b) 2-hydroxy-4-[3-hydroxy-3-(8,8-diméthyl-5,6,7,8-tétrahydro-2-naphtyl)-1-propynyl] benzoate de méthyle.

Dans un ballon, on introduit 7,95 g (22 mmoles) de 2-hydroxy-4-[3-oxo-3-(8,8-diméthyl-5,6,7,8-tétrahydro-2-naphtyl)-1-propynyl]benzoate de méthyle 150 ml de THF et 20 ml de méthanol. On ajoute par petites quantités 660 mg (17,4 mmoles) de borohydrure de sodium et agite à température ambiante pendant deux heures. On verse le milieu réactionnel dans l'eau glacée, neutralise avec de l'acide chlorhydrique, extrait avec de l'acétate d'éthyle, décante la phase organique, lave à l'eau, sèche sur sulfate de magnésium, évapore. Le résidu obtenu est purifié par chromatographie sur colonne de silice élué avec un mélange d'heptane et d'acétate d'éthyle (80-20). Après évaporation des solvants, on recueille 3,8 g (47,5%) de produit attendu sous forme d'une huile.

### (c) 2-hydroxy-4-[3-(8,8-diméthyl-5,6,7,8-tétrahydro-2-naphtyl)-1-propynyl]benzoate de méthyle.

De manière analogue à l'exemple 5(e) à partir de 4 g (11 mmoles) de l'ester méthylique précèdent, on obtient 1,13 g (29,5%) de 2-hydroxy-4-[3-(8,8-diméthyl-5,6,7,8-tétrahydro-2-naphtyl)-1-propynyl]benzoate de méthyle sous forme d'une huile orangée.

### (d) acide 2-hydroxy-4-[3-(8,8-diméthyl-5,6,7,8-tétrahydro-2-naphtyl)-1-propynyl] benzoïque.

De manière analogue à l'exemple 1(e) à partir de 1 g (2,9 mmoles) de l'ester méthylique précèdent, on obtient 370 mg (39%) d'acide 2-hydroxy-4-[3-(8,8-diméthyl-5,6,7,8-tétrahydro-2-naphtyl)-1-propynyl]benzoïque de point de fusion 18567°C.

### EXEMPLE 15

### Acide 2-hydroxy-4-[3-(5,5-diméthyl-5,6,7,8-tétrahydro-2-naphtyl)-1-propynyl]benzoïque.

### (a) 2-hydroxy-4-[3-oxo-3-(5,5-diméthyl-5,6,7,8-tétrahydro-2-naphtyl)-1-propynyl] benzoate de méthyle

De manière analogue à l'exemple 14(a) par réaction de 4,7 g (21 mmoles) de chlorure de 5,5-diméthyl-5,6,7,8-tétrahydro-2-naphtoyle avec 5,7 g (22,8 mmoles) de 2-hydroxy-4-triméthylsilyléthynylbenzoate de méthyle (préparé à l'exemple 5(a) du brevet EP 0 661 258), on obtient 5,14 g (68,5%) de l'ester méthylique attendu de point de fusion 89-90°C.

### (b) 2-hydroxy-4-[3-hydroxy-3-(5,5-diméthyl-5,6,7,8-tétrahydro-2-naphtyl)-1-propynyl] benzoate de méthyle.

De manière analogue à l'exemple 14(b) à partir de 2,4 g (6,6 mmoles) de 2-hydroxy-4-[3-oxo-3-(5,5-diméthyl-5,6,7,8-tétrahydro-2-naphtyl)-1-propynyl]benzoate de méthyle, on obtient 1,6 g (67%) de 2-hydroxy-4-[3-hydroxy-3-(5,5-diméthyl-5,6,7,8-tétrahydro-2-naphtyl)-1-propynyl]benzoate de méthyle sous forme d'une huile orangée.

### (c) acide 2-hydroxy-4-[3-hydroxy-3-(5,5-diméthyl-5,6,7,8-tétrahydro-2-naphtyl)-1-propynyl]benzoïque.

De manière analogue à l'exemple 1(e) à partir de 1,61 g (4,4 mmoles) de l'ester méthylique précédent, on obtient 1,2 g (77,4%) de l'acide attendu de point de fusion 141-2°C.

### (d) acide 2-hydroxy-4-[3-(5,5-diméthyl-5,6,7,8-tétrahydro-2-naphtyl)-1-propynyl] benzoïque.

De manière analogue à l'exemple 5(e) à partir de 580 mg (1,65 mmoles) d'acide 2-hydroxy-4-[3-hydroxy-3-(5,5-diméthyl-5,6,7,8-tétrahydro-2-naphtyl)-1-propynyl]benzoïque, on obtient 470 mg (85%) d'acide 2-hydroxy-4-[3-(5,5-diméthyl-5,6,7,8-tétrahydro-2-naphtyl)-1-propynyl]benzoïque de point de fusion 152-3°C.

### EXEMPLE 16

### 4-[3-(5,6,7,8-tétrahydro-5,5,8,8-tétraméthyl-2-naphtyl)-1-propynyl]benzoate d'ethyle.

### (a) méthoxyallène.

Dans un tricol et sous argon, on introduit 210 ml (2,5 moles) de propargyl méthylether et 12 g (0,11 mole) de *tert*-butylate de potassium. On chauffe à reflux pendant trois heures et distille le milieu réactionnel à pression atmosphérique et recueille la fraction passant à 51°C. On obtient 153,5 g (88%) du produit attendu sous forme d'une huile incolore.

### (b) 3-(5,6,7,8-tétrahydro-5,5,8,8-tétraméthyl-2-naphtyl)-1-propyne.

Dans un réacteur de quatre litres et sous courant d'azote, on introduit 20 g (0,82 mole) de magnésium activé par 0,1 ml de dibromoethane. On ajoute goutte à goutte une solution de 200 g (0,75 mole) de 2-bromo-5,6,7,8-tétrahydro-5,5,8,8-tétraméthylnaphtalene de manière à maintenir le reflux du THF et agite à 50°C pendant deux heures. On refroidit ensuite le milieu réactionnel à -5°C et ajoute 1,2 g (8,2 mmoles) de CuBr et introduit goutte à goutte une solution de 58 g (0,82 mole) de methoxyallene dans 100 ml de THF. On agite une heure à -5°C puis laissse remonter à température ambiante et agite deux heures. On verse le milieu réactionnel dans une solution saturée de chlorure d'ammonium, extrait avec de l'acétate d'éthyle, décante la phase organique, sèche sur sulfate de magnésium, évapore. On distille l'huile obtenu sous 0,02 mm de Hg et recueille la fraction passant à 95-100°C. On obtient 79 g (47%) du produit attendu sous forme d'une huile incolore.

### (c) 4-[3-(5,6,7,8-tétrahydro-5,5,8,8-tétraméthyl-2-naphtyl)-1-propynyl]benzoate d'éthyle.

Dans un tricol et sous courant d'azote, on introduit 7,4 g (32,7 mmoles) de 3-(5,6,7,8-tétrahydro-5,5,8,8-tétraméthyl-2-naphtyl)-1-propyne 8,2 g (29,7 mmoles) de 4-iodobenzoate d'éthyle et 50 ml de triéthylamine. On dégaze le milieu réactionnel par barbotage d'azote, et introduit 360 mg (0,5 mmole) de Bis(triphenylphosphine) palladium(ll)chlorure, 130 mg d'iodure de cuivre et agite à température ambiante pendant huit heures. On évapore à sec le milieu réactionnel, reprend par l'acétate d'éthyle et l'acide chlorhydrique (1N), décante la phase organique, sèche sur sulfate de magnésium, évapore.Le résidu est purifié par chromatographie sur colonne de silice élué avec un mélange d'heptane et d'acétate d'éthyle (70-30). Après évaporation des solvants, on recueille un solide que l'on triture dans l'heptane, filtre, sèche. On recueille 9,3 g (84%) de l'ester éthylique attendu de point de fusion 59-60°C.

### EXEMPLE 17

### 4-[3-(5,6,7,8-tétrahydro-5,5,8,8-tétraméthyl-2-naphtyl)-1-propynyl]benzamide.

### (a) acide 4-[3-(5,6,7,8-tétrahydro-5,5,8,8-tétraméthyl-2-naphtyl)-1-propynyl] benzoïque.

De manière analogue à l'exemple 16(c) par réaction de 8 g (35,3 mmoles) de 3-(5,6,7,8-tétrahydro-5,5,8,8-tétraméthyl-2-naphtyl)-1-propyne avec 8 g (32,1 mmoles) d'acide 4-iodobenzoïque, on obtient 10,4 g (94%) de l'acide attendu de point de fusion 167-8°C.

### (b) chlorure de 4-[3-(5,6,7,8-tétrahydro-5,5,8,8-tétraméthyl-2-naphtyl)-1-propynyl] benzoyle.

Dans un ballon et sous courant d'azote, on introduit 2,9 g (8,3 mmoles) d'acide 4-[3-(5,6,7,8-tétrahydro-5,5,8,8-tétraméthyl-2-naphtyl)-1-propynyl] benzoïque 100 ml de dichlorométhane et ajoute goutte à goutte 2,4 ml (12,1 mmoles) de dicyclohexylamine. On agite à température ambiante pendant une heure, ajoute goutte à goutte 1,2 ml (11,7 mmoles) de chlorure de thionyle et agite une heure. On évapore à sec le milieu réactionnel, reprend par l'éther éthylique, filtre le sel de dicyclohexylamine, évapore. On recueille 3 g (100%) de chlorure d'acide brut qui sera utilisé tel quel pour la suite de la synthèse.

### (c) 4-[3-(5,6,7,8-tétrahydro-5,5,8,8-tétraméthyl-2-naphtyl)-1-propynyl]benzamide.

Dans un ballon, on introduit 3 g (8,3 mmoles) du chlorure d'acide précédent dissous dans 100 ml de THF et ajoute 1 ml (9,1 mmoles) d'ammoniaque (32%) et agite à température ambiante une heure. On verse le milieu réactionnel dans l'eau, extrait avec du dichlorométhane, décante la phase organique, lave à l'eau, sèche sur sulfate de magnésium, évapore. Le solide obtenu est trituré dans l'heptane, filtré, séché. On recueille 2,5 g (87%) de 4-[3-(5,6,7,8-tétrahydro-5,5,8,8-tétraméthyl-2-naphtyl)-1-propynyl]benzamide de point de fusion 207-8°C.

### EXEMPLE 18

### N-ethyl-4-[3-(5,6,7,8-tétrahydro-5,5,8-tétraméthyl-2-naphtyl)-1-propynyl] benzamide.

De manière analogue à l'exemple 17 (b) par réaction de 3g (8,3 mmoles) de chlorure de 4-[3-(5,6,7,8-tétrahydro-5,5,8,8-tétraméthyl-2-naphtyl)-1-propynyl] benzoyle avec 650 µl (10 mmoles) d'ethylamine (70%), on obtient 1,84 g (59,4%) d'amide éthylique attendu de point de fusion 128-9°C.

### EXEMPLE 19

### morpholide de l'acide 4-[3-(5,6,7,8-tétrahydro-5,5,8,8-tétraméthyl-2-naphtyl)-1-propynyl]benzoique.

De manière analogue à l'exemple 17 (b) par réaction de 1,25 g (3,4 mmoles) de chlorure de 4-[3-(5,6,7,8-tétrahydro-5,5,8,8-tétraméthyl-2-naphtyl)-1-propynyl] benzoyle avec 320 µL (3,8 mmoles) de morpholine, on obtient 430 mg (31%) du morpholide de l'acide 4-[3-(5,6,7,8-tétrahydro-5,5,8,8-tétraméthyl-2-naphtyl)-1-propynyl]benzoïque de point de fusion 98-9°C.

### EXEMPLE 20

### N-(4-hydroxyphenyl)-4-[3-(5,6,7,8-tétrahydro-5,5,8,8-tétraméthyl-2-naphtyl)-1-propynyl]benzamide.

De manière analogue à l'exemple 17 (b) par réaction de 3 g (8,3 mmoles) de chlorure de 4-[3-(5,6,7,8-tétrahydro-5,5,8,8-tétraméthyl-2-naphtyl)-1-propynyl] benzoyle avec 1 g (9,2 mmoles) de 4-aminophenol, on obtient 1,94 g (54%) de N-(4-hydroxyphenyl)-4-[3-(5,6,7,8-tétrahydro-5,5,8,8-tétraméthyl-2-naphtyl)-1-propynyl]benzamide de point de fusion 159-60°C.

### EXEMPLE 21

### 4-[3-(5,6,7,8-tétrahydro-5,5,8,8-tétraméthyl-2-naphtyl)-1-propynyl] benzenemethanol.

De manière analogue à l'exemple 16(c) par réaction de 3,67 g (16,2 mmoles) de 3-(5,6,7,8-tétrahydro-5,5,8,8-tétraméthyl-2-naphtyl)-1-propyne avec 3,45 g (14,8 mmoles) de 4-iodobenzènemethanol, on obtient 4,9 g (100%) de 4-[3-(5,6,7,8-tétrahydro-5,5,8,8-tétraméthyl-2-naphtyl)-1-propynyl] benzenemethanol sous forme d'une huile visqueuse orange.

RMN 1H (CDCl3, 250 MHz) 1,27 (6H,s), 1,29 (6H,s), 1,68 (4H,s), 3,77 (2H,s), 4,67 (2H,d), 7,17 (1H Ar,dd), 7,27 (2H Ar,d), 7,30 (2H Ar,d), 7,42 (2H Ar,c).

### EXEMPLE 22

### 4-[3-(5,6,7,8-tétrahydro-5,5,8,8-tétraméthyl-2-naphtyl)-1-propynyl] benzaldehyde

Dans un ballon, on introduit 1,3 g (4 mmoles) de 4-[3-(5,6,7,8-tétrahydro-5,5,8,8-tétraméthyl-2-naphtyl)-1-propynyl] benzenemethanol 20 ml de dichloromethane et ajoute 3,4 g (39,2 mmoles) d'oxyde de manganèse. On agite à température ambiante pendant huit heures. On ajoute au milieu réactionnel du sulfate de magnésium, filtre et évapore le filtrat. Le résidu obtenu est purifié par chromatographie sur colonne de silice élué avec un mélange d'acétate d'éthyle et d'heptane (10-90). Après évaporation des solvants, on recueille 930 mg (29,5%) de 4-[3-(5,6,7,8-tétrahydro-5,5,8,8-tétraméthyl-2-naphtyl)-1-propynyl] benzaldehyde sous forme d'une huile jaune.

RMN 1H (CDCl3, 250 MHz) 1,28 (6H,s), 1,30 (6H,s), 1,69 (4H,s), 3,81 (2H,s), 7,20 (1H Ar,dd), 7,31 (2H Ar,c) 7,59 (2H Ar,d), 7,80 (2H Ar,d), 9,99 (1H,s).

### EXEMPLE 23

### 4-[3-(5,6,7,8-tétrahydro-5,5,8,8-tétraméthyl-2-naphtyl)-1-propynyl]phenol,

De manière analogue à l'exemple 16(c) par réaction de 1,13 g (5 mmoles) de 3-(5,6,7,8-tétrahydro-5,5,8,8-tétraméthyl-2-naphtyl)-1-propyne avec 1 g (4,5 mmoles) de 4-iodophenol, on obtient 1,28 g (88%) de 4-[3-(5,6,7,8-tétrahydro-5,5,8,8-tétraméthyl-2-naphtyl)-1-propynyl]phenol sous forme d'une huile visqueuse rouge-orangée.

RMN 1H (CDCl3, 250 MHz) 1,27 (6H,s), 1,29 (6H,s), 1,68 (4H,s), 3,75 (2H,s),5,05 (1H,s), 6,76 (2H,d), 7,20 (1H Ar,dd), 7,25 (1H,d), 7,31 (3H Ar,c).

### EXEMPLE 24

### 4-[3-(5,6,7,8-tétrahydro-5,5,8,8-tétraméthyl-2-naphtyl)-1-propynyl]toluene.

De manière analogue à l'exemple 16(c) par réaction de 1,62 g (7,15 mmoles) de 3-(5,6,7,8-tétrahydro-5,5,8,8-tétraméthyl-2-naphtyl)-1-propyne avec 1,42 g (6,5 mmoles) de 4-iodotoluène, on obtient 1,29 g (63%) de 4-[3-(5,6,7,8-tétrahydro-5,5,8,8-tétraméthyl-2-naphtyl)-1-propynyl]toluene sous forme d'une huile jaunatre.

RMN 1H (CDCl3, 250 MHz) 1,27 (6H,s), 1,29 (6H,s), 1,69 (4H,s),2,33 (3H,s) 3,81 (2H,s), 7,20 (1H Ar,dd), 7,31 (2H Ar,c) 7,59 (2H Ar,d), 7,80 (2H Ar,d).

### EXEMPLE 25

### 4-[3-(5,6,7,8-tétrahydro-5,5,8,8-tétraméthyl-2-naphtyl)-1-propynyl]benzoate d'hexyle.

Dans un ballon, on introduit 2 g (5,8 mmoles) d'acide 4-[3-(5,6,7,8-tétrahydro-5,5,8,8-tétraméthyl-2-naphtyl)-1-propynyl]benzoïque 30 ml de DMF 2 gouttes de 15-crown-5 et 2 g (23,8 mmoles) de bicarbonate de sodium. On ajoute 3,1 ml (20,8 mmoles) de 1-iodohexane et agite à température ambiante pendant 24 heures. On verse le milieu réactionnel dans l'eau, extrait avec de l'acétate d'éthyle, décante la phase organique, lave à l'eau, sèche sur sulfate de magnésium, évapore. On recueille 2 g (80%) de l'ester hexylique attendu sous forme d'une huile légèrement rouge.

RMN 1H (CDCI3, 250 MHz) 0,9 (3H,t), 1,27 (6H,s), 1,29 (6H,s), 1,34 (6H,c), 1,66 (4H,s), 1,72 (2H,m), 3,81 (2H,s), 4,30 (2H,t), 7,20 (1H Ar,dd), 7,31 (2H Ar,c) 7,50 (2H Ar d) 7,95 (2H Ar,d).

### EXEMPLE 26

### N-hydroxy-2-hydroxy-4-[3-(5,6,7,8-tétrahydro-5,5,8,8-tétraméthyl-2-naphtyl)-1-propynyl]benzamide.

### (a) N-hydroxy-2-hydroxy-4-iodobenzamide.

Dans un tricol et sous courant d'azote, on introduit 23,3 g (84 mmoles) de 2-hydroxy-4-iodobenzoate de methyle et 360 ml d'une solution d'hydroxyde de sodium (1N). On ajoute 8 g (113 mmoles) de chlorhydrate d'hydroxylamine et agite à température ambiante deux heures. On ajuste le milieu réactionnel à pH 7-8 avec de l'acide chlorhydrique concentré, filtre le solide. On dissout le solide dans l'acétate d'éthyle, lave à l'eau, décante la phase organique, sèche sur sulfate de magnésium, évapore. Le résidu est trituré dans l'heptane, filtré, séché. On recueille 17,6 g (71,5%) du produit attendu de point de fusion 195-6°C.

### (b) N-hydroxy-2-hydroxy-4-[3-(5,6,7,8-tétrahydro-5,5,8,8-tétraméthyl-2-naphtyl)-1-propynyl]benzamide.

De manière analogue à l'exemple 16(c) par réaction de 4 g (17,7 mmoles) de 3-(5,6,7,8-tétrahydro-5,5,8,8-tétraméthyl-2-naphtyl)-1-propyne avec 4,1 g (14,7 mmoles) de N-hydroxy-2-hydroxy-4-iodobenzamide, on obtient 550 mg (10%) de N-hydroxy-2-hydroxy-4-[3-(5,6,7,8-tétrahydro-5,5,8,8-tétraméthyl-2-naphtyl)-1-propynyl]benzamide de point de fusion 119-20°C.

### EXEMPLE 27

### Acide 2-methyl-4-[3-(5,6,7,8-8-tétrahydro-5,5,8-8-tétraméthyl-2-naphtyl)-1-propynyl)benzoïque.

De manière analogue à l'exemple 16(c) par réaction de 10 g (44,2 mmoles) de 3-(5,6,7,8-tétrahydro-5,5,8,8-tétraméthyl-2-naphtyl)-1-propyne avec 6,5 g (29,5 mmoles) d'acide 4-bromo-2-methylbenzoïque, on obtient 1,18 g (11%) d'acide 2-methyl-4-[3-(5,6,7,8-tétrahydro-5,5,8,8-tétraméthyl-2-naphtyl)-1-propynyl]benzoïque de point de fusion 149-50°C.

### EXEMPLE 28

### Acide 3-methyl-4-[3-(5,6,7,8-tétrahydro-5,5,8,8-tétraméthyl-2-naphtyl)-1-propynyl]benzoïque.

De manière analogue à l'exemple 16(c) par réaction de 10 g (44,2 mmoles) de 3-(5,6,7,8-tétrahydro-5,5,8,8-tétraméthyl-2-naphtyl)-1-propyne avec 6,5 g (29,5 mmoles) d'acide 4-bromo-3-methylbenzoïque, on obtient 1,1 g (11%) d'acide 3-methyl-4-[3-(5,6,7,8-tétrahydro-5,5,8,8-tétraméthyl-2-naphtyl)-1-propynyl]benzoïque de point de fusion 196-7°C.

### EXEMPLE 29

### Acide 6-[3-(5,5,8,8-tetramethyl-5,6,7,8-tetrahydro-2-naphtyl)propa-1,2-dienyl]nicotinique.

### (a) 6-[3-(5,5,8,8-tetramethyl-5,6,7,8-tetrahydro--2-naphtyl)propa-1,2-dienyl]nicotinate de méthyle.

De manière analogue à l'exemple 16(c) par réaction de 920 mg (4,1 mmoles) de (5,6,7,8-tétrahydro-5,5,8,8-tétraméthyl-2-naphtyl)-1-propynyle avec 1,1 g (4,2 mmoles) de 4-iodonicotinate de méthyle, on obtient 260 mg (18%) de 6-[3-(5,5,8,8-tetramethyl-5,6,7,8-tetrahydro--2-naphtyl)propa-1,2-dienyl]nicotinate de méthyle sous forme d'une huile orangée.

RMN 1H (CDCl3, 250 MHz) 1,34 (12H,s), 1,75 (4H,s), 3,87 (3H,s), 6,56 (1H,d), 6,92 (1H,d), 7,19 (1H,d), 7,29 (1H,dd), 7,42 (2H,t), 7,50 (1H,d), 9,10 (1H,s).

### (b) acide 6-[3-(5,5,8,8-tetramethyl-5,6,7,8-tetrahydro--2-naphtyl)propa-1,2-dienyl] 3-nicotinique

Dans un ballon, on introduit 370 mg (1 mmole) de l'ester méthylique précédent 10 ml de THF et 5 ml d'une solution de soude méthanolique (2N). On chauffe à 40°C pendant une heure, évapore à sec, reprend par l'eau, ajuste à pH 5 avec de l'acide chlorhydrique, extrait avec de l'acétate d'éthyle, décante la phase organique, sèche sur sulfate de magnésium, évapore. Le résidu est trituré dans l'heptane, filtré, séché. On recueille 240 mg (66%) d'acide attendu de point de fusion 224-5°C.

### EXEMPLE 30

### Acide 4-[3-(5,5,8,8-tetramethyl-5,6,7,8-tetrahydro--2-naphtyl)propa-1,2-dienyl]benzoïque.

Dans un ballon, on introduit 6,02 g (16 mmoles) de 4-[3-(5,6,7,8-tétrahydro-5,5,8,8-tétraméthyl-2-naphtyl)-1-propynyl]benzoate d'éthyle 5 ml de méthanol 5 ml de THF et 50 ml d'heptane. On ajoute 850 mg d'hydroxyde de sodium et chauffe à reflux pendant une heure. On verse le milieu réactionnel dans l'eau, ajuste à pH 1 avec de l'acide chlorhydrique extrait avec de l'acétate d'éthyle, décante la phase organique, sèche sur sulfate de magnésium, évapore. Le résidu obtenu est trituré dans l'alcool éthylique, filtré, séché. On recueille 1,37 g (24,5%) d'acide 4-[3-(5,5,8,8-Tetramethyl-5,6,7,8-tetrahydro--2-naphtyl)propa-1,2-dienyl]benzoïque.de point de fusion 227-8°C.

### EXEMPLE 31

### Acide 2-hydroxy-4-[3-(5,6,7,8-tétrahydro-5,5,8,8-tétraméthyl-2-naphtyl)-1-butynyl]benzoïque.

De manière analogue à l'exemple 5(e) à par partir de 1 g (2,55 mmoles) d'acide 2-hydroxy-4-[3-hydroxy-3-(5,6,7,8-tétrahydro-5,5,8,8-tétraméthyl-2-naphtyl)-1-butynyl]benzoïque (préparé à l'exemple 30 du brevet EP 0 661 258), on obtient 450 mg (47%) d'acide 2-hydroxy-4-[3-(5,5,8,8-tetramethyl-5,6,7,8-tetrahydro--2-naphtyl)-1-butynyl]benzoïque de point de fusion 191-2°C.

### EXEMPLE 32

Dans cet exemple, on a illustré diverses formulations concrètes à base des composés selon l'invention.

### A- VOIE ORALE

(a) Comprimé de 0,2 g
   - Composé de l'exemple 2 0,001 g
   - Amidon 0,114 g
   - Phosphate bicalcique 0,020 g
   - Silice 0,020 g
   - Lactose 0,030 g
   - Talc 0,010 g
   - Stéarate de magnésium 0,005 g
(b) Suspension buvable en ampoules de 5 ml
   - Composé de l'exemple 6 0,001 g
   - Glycérine 0,500 g
   - Sorbitol à 70% 0,500 g
   - Saccharinate de sodium 0,010 g
   - Parahydroxybenzoate de méthyle 0,040 g
   - Arome qs
   - Eau purifiée qsp 5 ml
(c) Comprimé de 0,8 g
   - Composé de l'exemple 5 0,500 g
   - Amidon prégélatinisé 0,100 g
   - Cellulose microcristalline 0,115 g
   - Lactose 0,075 g
   - Stéarate de magnésium 0,010 g
(d) Suspension buvable en ampoules de 10 ml
   - Composé de l'exemple 2 0,05 g
   - Glycérine 1,000g
   - Sorbitol à 70% 1,000g
   - Saccharinate de sodium 0,010 g
   - Parahydroxybenzoate de méthyle 0,080 g
   - Arome qs
   - Eau purifiée qsp 10 ml

### B- VOIE TOPIQUE

(a) Onguent
   - Composé de l'exemple 9 0,020 g
   - Myristate d'isopropyle 81,700 g
   - Huile de vaseline fluide 9,100 g
   - Silice ("Aérosil 200" vendue par DEGUSSA) 9,180 g
(b) Onguent
   - Composé de l'exemple 7 0,300 g
   - Vaseline blanche codex 100 g
(c) Crème Eau-dans-Huile non ionique
   - Composé de l'exemple 25 0,100 g
   - Mélange d'alcools de lanoline émulsifs, de cires et d'huiles ("Eucerine anhydre" vendu par BDF) 39,900 g
   - Parahydroxybenzoate de méthyle 0,075 g
   - Parahydroxybenzoate de propyle 0,075 g
   - Eau déminéralisée stérile qsp 100 g
(d) Lotion
   - Composé de l'exemple 8 0,100 g
   - Polyéthylène glycol (PEG 400) 69,900 g
   - Ethanol à 95% 30,000 g
(e) Onguent hydrophobe
   - Composé de l'exemple 20 0,300 g
   - Mirystate d'isopropyle 36,400 g
   - Huile de silicone ("Rhodorsil 47 V 300" vendu par RHONE-POULENC) 36,400 g
   - Cire d'abeille 13,600 g
   - Huile de silicone ("Abil 300.000 cst" vendu par GOLDSCHMIDT) 100g
(f) Crème Huile-dans-Eau non ionique
   - Composé de l'exemple 30 0,500 g
   - Alcool cétylique 4,000 g
   - Monostéarate de glycérole 2,500 g
   - Stéarate de PEG 50 2,500 g
   - Beurre de karité 9,200 g
   - Propylène glycol 2,000 g
   - Parahydroxybenzoate de méthyle 0,075 g
   - Parahydroxybenzoate de propyle 0,075 g
   - Eau déminéralisée stérile 100 g

## Revendications

1. Composés propynyl biaromatiques, caractérisés par le fait qu'ils répondent à la formule générale (I) suivante : 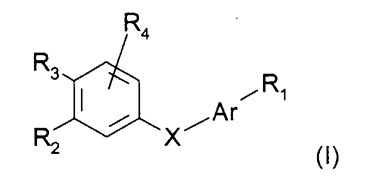 dans laquelle:
- R₁ représente
(i) le radical -CH₃
(ii) le radical -CH₂-O-R₆
(iii) le radical -O-R₆
(iv) le radical -CO-R₇
R₆ et R₇ ayant les significations données ci-après,
- Ar représente un radical choisi parmi les radicaux de formules (a)-(e) suivantes: 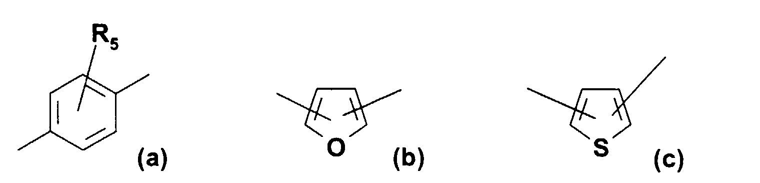 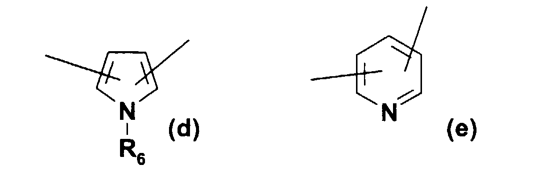 R₅ et R₆ ayant les significations données ci-après,
- X représente un radical de formule: 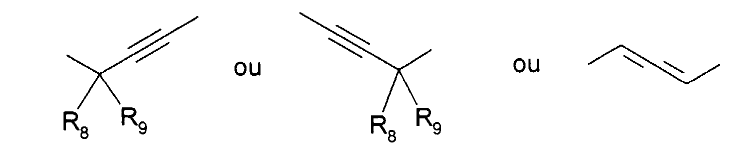 R₈ et R₉ ayant les significations données ci-après,
- R₂ et R₃, identiques ou différents, représentent
(i) un atome d'hydrogène,
(ii) un radical alkyle linéaire ou ramifié ayant de 1 à 20 atomes de carbone,
(iii) un radical -OR₆,
(iv) un radical -SR₆,
R₆ ayant la signification donnée ci-après,
étant entendu que R₂ et R₃ pris ensemble peuvent former avec le cycle aromatique adjacent un cycle à 5 ou 6 chainons éventuellement substitué par des groupes méthyle et/ou éventuellement interrompu par un atome d'oxygène ou de soufre,
et étant entendu que R₂ et R₃ ne peuvent pas avoir en même temps les significations (i), (iii) et (iv) mentionnées ci-dessus.
- R₄ et R₅, identiques ou différents, représentent un atome d'hydrogène, un atome d'halogène, un radical alkyle linéaire ou ramifié ayant de 1 à 20 atomes de carbone, un radical -OR₆,
étant entendu que lorsque R₄ est un radical hydroxyle alors R₂ et R₃ forment avec le cycle aromatique adjacent un cycle à 5 ou 6 chaînons éventuellement substitué par des groupes méthyle et/ou éventuellement interrompu par un atome d'oxygène ou de soufre,
- R₆ représente un atome d'hydrogène, un radical alkyle ayant de 1 à 12 atomes de carbone ou un radical -COR₁₀
R₁₀ ayant la signification donnée ci-après,
- R₇ représente:
(a) un atome d'hydrogène
(b) un radical alkyle inférieur
(c) un radical de formule: 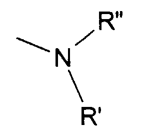
R' et R" ayant la signification donnée ci-après,
(d) un radical -OR₁₁
(e) un radical -NHOR₆,
R₁₁ ayant la signification donnée ci-après,
- R₈ et R₉, pris séparément, soit ont simultanément la même signification : un atome d'hydrogène, soit l'un représente un atome d'hydrogène et l'autre un radical alkyle ayant de 1 à 12 atomes de carbone ou, pris ensemble, forment un cycle -Y-(CH₂)n-Y-, avec Y représentant un atome d'oxygène ou de soufre et avec n égal à 2 ou 3,
- R₁₀ représente un radical alkyle ayant de 1 à 12 atomes de carbone,
- R₁₁ représente un atome d'hydrogène, un radical alkyle linéaire ou ramifié, ayant de 1 à 20 atomes de carbone, un radical alkényl, un radical mono ou polyhydroxyalkyle, un radical aryle ou aralkyle, éventuellement substitué(s) ou un reste de sucre ou un reste d'amino acide ou de peptide,
- R' et R", identiques ou différents, représentent un atome d'hydrogène, un radical alkyl ayant de 1 à 12 atomes de carbone, un radical mono ou polyhydroxyalkyle, un radical aryle éventuellement substitué ou un reste d'amino acide ou de sucre ou encore pris ensemble forment un hétérocycle,
ainsi que leurs sels et leurs isomères optiques et géométriques.

2. Composés, caractérisés par le fait qu'ils répondent à la formule générale (II) suivante : 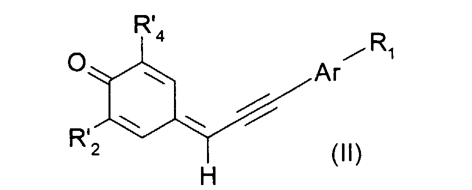 dans laquelle R₁ et Ar ont les mêmes significations que pour la formule générale (I) définie à la revendication 1 et R'₂ et R'₄, identiques ou différents, représentent un radical alkyle linéaire ou ramifié ayant de 1 à 20 atomes de carbone.

3. Composés selon la revendication 1 ou 2, caractérisés par le fait qu'ils se présentent sous forme de sels d'un métal alcalin ou alcalino-terreux, ou encore de zinc ou d'une amine organique.

4. Composés selon la revendication 1, caractérisés par le fait qu'ils sont pris, seuls ou en mélanges, dans le groupe constitué par :
- Acide 4-[3-(3,5-di-tert-butyl-4-hydroxyphenyl)-1-propynyl] benzoïque.
- Acide 2-hydroxy-4-[3-(3,5-di-tert-butyl-4-hydroxyphenyl)-1-propynyl] benzoïque.
- Acide 4-[3-(5,6,7,8-tétrahydro-5,5,8,8-tétraméthyl-2-naphtyl)-1-propynyl] benzoïque.
- 2-hydroxy-4-[3-(5,6,7,8-tétrahydro-5,5,8,8-tétraméthyl-2-naphtyl)-1-propynyl]benzoate de méthyle.
- Acide 2-hydroxy-4-[3-(5,6,7,8-tétrahydro-5,5,8,8-tétraméthyl-2-naphtyl)-1-propynyl]benzoïque.
- Acide 2-hydroxy-4-[3-(3-hydroxy-5,6,7,8-tétrahydro-5,5,8,8-tétraméthyl-2-naphtyl)-1-propynyl]benzoïque.
- 2-hydroxy-4-[3-(5,6,7,8-tétrahydro-5,5,8,8-tétraméthyl-2-naphtyl)-1-propynyl] benzèneméthanol.
- 2-hydroxy-4-[3-(5,6,7,8-tétrahydro-5,5,8,8-tétraméthyl-2-naphtyl)-1-propynyl] benzoate de diéthanolamine.
- 2-hydroxy-4-[3-(5,6,7,8-tétrahydro-5,5,8,8-tétraméthyl-2-naphtyl)-1-propynyl] benzoate de lithium.
- Acide 4-[3-(5,6,7,8-tétrahydro-5,5,8,8-tétraméthyl-2-naphtyl)-2-propynyl] benzoïque.
- Acide 2-hydroxy-4-[3-(4,4-dimethylthiochromane-6-yl)-1-propynyl]benzoïque.
- Acide 2-hydroxy-4-[3-(8,8-diméthyl-5,6,7,8-tétrahydro-2-naphtyl)-1-propynyl] benzoïque.
- Acide 2-hydroxy-4-[3-(5,5-diméthyl-5,6,7,8-tétrahydro-2-naphtyl)-1-propynyl] benzoïque.
- 4-[3-(5,6,7,8-tétrahydro-5,5,8,8-tétraméthyl-2-naphtyl)-1-propynyl]benzoate d'ethyle.
- 4-[3-(5,6,7,8-tétrahydro-5,5,8,8-tétraméthyl-2-naphtyl)-1-propynyl] benzamide.
- N-ethyl-4-[3-(5,6,7,8-tétrahydro-5,5,8,8-tétraméthyl-2-naphtyl)-1-propynyl] benzamide.
- Morpholide de l'acide 4-[3-(5,6,7,8-tétrahydro-5,5,8,8-tétraméthyl-2-naphtyl)-1-propynyl]benzoïque.
- N-(4-hydroxyphenyl)-4-[3-(5,6,7,8-tétrahydro-5,5,8,8-tétraméthyl-2-naphtyl)-1-propynyl]benzamide.
- 4-[3-(5,6,7,8-tétrahydro-5,5,8,8-tétraméthyl-2-naphtyl)-1-propynyl] benzaldehyde.
- 4-[3-(5,6,7,8-tétrahydro-5,5,8,8-tétraméthyl-2-naphtyl)-1-propynyl]phenol.
- [3-(5,6,7,8-tétrahydro-5,5,8,8-tétraméthyl-2-naphtyl)-1-propynyl]benzene methanol.
- 4-[3-(5,6,7,8-tétrahydro-5,5,8,8-tétraméthyl-2-naphtyl)-1-propynyl]toluene.
- 4-[3-(5,6,7,8-tétrahydro-5,5,8,8-tétraméthyl-2-naphtyl)-1-propynyl]benzoate d'hexyle.
- N-hydroxy-4-[3-(5,6,7,8-tétrahydro-5,5,8,8-tétraméthyl-2-naphtyl)-1-propynyl] benzamide.
- N-hydroxy-2-hydroxy-4-[3-(5,6,7,8-tétrahydro-5,5,8,8-tétraméthyl-2-naphtyl)-1-propynyl]benzamide.
- Acide 2-methyl-4-[3-(5,6,7,8-tétrahydro-5,5,8,8-tétraméthyl-2-naphtyl)-1-propynyl]benzoïque.
- Acide 3-methyl-4-[3-(5,6,7,8-tétrahydro-5,5,8,8-tétraméthyl-2-naphtyl)-1-propynyl]benzoïque.
- Acide 6-[3-(5,5,8,8-tetramethyl-5,6,7,8-tetrahydro--2-naphtyl)propa-1,2-dienyl]nicotinique.
- Acide 4-[3-(5,5,8,8-tetramethyl-5,6,7,8 -tetrahydro--2-naphtyl)propa-1,2-dienyl]benzoïque.
- Acide 2-hydroxy-4-[3-(5,5,8,8-tetramethyl-5,6,7,8-tetrahydro--2-naphtyl) propa-1,2-dienyl]benzoïque.
- Acide 2-hydroxy-4-[3-(5,6,7,8-tétrahydro-5,5,8,8-tétraméthyl-2-naphtyl)-1-butynyl]benzoïque.
- Acide 5-[3-(5,6,7,8-tétrahydro-5,5,8,8-tétraméthyl-2-naphtyl)-1-propynyl]-2-pyridinecarboxylique.
- Acide 4-[3-(3,5,5,8,8-pentaméthyl-5,6,7,8-tétrahydro-2-naphtyl)-1-propynyl] benzoïque.
- Acide 2-[3-(5,6,7,8-tétrahydro-5,5,8,8-tétraméthyl-2-naphtyl)-1-propynyl]-4-thiophenecarboxylique.
- Acide 2-[3-(3,5,5,8,8-pentaméthyl-5,6,7,8-tétrahydro-2-naphtyl)-1-propynyl]-4-thiophenecarboxylique.
- Acide 2-hydroxy-4-[3-(3-*tert*-butyl-4-methoxyphenyl)-1-propynyl]benzoïque.
- Acide 2-hydroxy-4-[3-(3-*tert*-butyl-4-hydroxyphenyl)-1-propynyl]benzoïque.

5. Composés selon la revendication 2, caractérisés par le fait qu'ils sont pris, seuls ou en mélanges, dans le groupe constitué par :
- Acide 4-[3-(3,5-di-tert-butyl-4-oxo-2,5-cyclohexadiene-1-ylidene)-1-propynyl]benzoïque.
- Acide 2-hydroxy-4-[3-(3,5-di-tert-butyl-4-oxo-2,5-cyclohexadiene-1-ylidene)-1-propynyl]benzoïque.

6. Composés selon la revendication 1 ou 2, caractérisés par le fait qu'ils présentent au moins l'une des, et de préférence toutes les, caractéristiques suivantes :
- R₁ représente le radical -CO-R₇,
- Ar représente les radicaux de formule (a) ou (e).

7. Composés selon l'une des revendications 1, 3 ou 6, caractérisés par le fait qu'ils présentent une formule (I) telle que définié à la revendication 1 dans laquelle R₈ et R₉, pris séparément, soit représentent des atomes d'hydrogène, soit l'un représente un atome d'hydrogène et l'autre un radical alkyle ayant de 1 à 12 atomes de carbone.

8. Composés de formule (I) ou (II) selon l'une quelconque des revendications précédentes pour une utilisation comme médicament.

9. Composés selon la revendication 8 pour une utilisation comme médicament destiné au traitement des affections dermatologiques liées à un désordre de la kératinisation portant sur la différenciation et sur la prolifération notamment pour traiter les acnés vulgaires, comédoniennes, polymorphes, rosacées, les acnés nodulokystiques, conglobata, les acnés séniles, les acnés secondaires telles que l'acné solaire, médicamenteuse ou professionnelle; pour traiter d'autres types de troubles de la kératinisation, notamment les ichtyoses, les états ichtyosiformes, la maladie de Darier, les kératodermies palmoplantaires, les leucoplasies et les états leucoplasiformes, le lichen cutané ou muqueux (buccal); pour traiter d'autres affections dermatologiques liées à un trouble de la kératinisation avec une composante inflammatoire et/ou immuno-allergique et, notamment, toutes les formes de psoriasis qu'il soit cutané, muqueux ou unguéal, et même le rhumatisme psoriatique, ou encore l'atopie cutanée, telle que l'eczéma ou l'atopie respiratoire ou encore l'hypertrophie gingivale; les composés peuvent également être utilisés dans certaines affections inflammatoires ne présentant pas de trouble de la kératinisation; pour traiter toutes les proliférations dermiques ou épidermiques qu'elles soient bénignes ou malignes, qu'elles soient ou non d'origine virale telles que les verrues vulgaires, les verrues planes et l'épidermodysplasie verruciforme, les papillomatoses orales ou florides et les proliférations pouvant être induites par les ultra-violets notamment dans le cas des épithélioma baso et spinocellulaires; pour traiter d'autres désordres dermatologiques tels que les dermatoses bulleuses et les maladies du collagène; pour traiter certains troubles ophtalmologiques, notamment les coméopathies; pour réparer ou lutter contre le vieillissement de la peau, qu'il soit photoinduit ou chronologique ou pour réduire les pigmentations et les kératoses actiniques, ou toutes pathologies associées au vieillissement chronologique ou actinique; pour prévenir ou guérir les stigmates de l'atrophie épidermique et/ou dermique induite par les corticostéroïdes locaux ou systémiques, ou tout autre forme d'atrophie cutanée, pour prévenir ou traiter les troubles de la cicatrisation ou pour prévenir ou réparer les vergetures; pour favoriser la cicatrisation, pour lutter contre les troubles de la fonction sébacée tels que l'hyperséborrhée de l'acné ou la séborrhée simple; pour le traitement ou la prévention des états cancéreux ou précancéreux, plus particulièrement les leucémies promyélocytaires; pour le traitement d'affections inflammatoires telles que l'arthrite, pour le traitement de toute affection d'origine virale au niveau cutané ou général; pour la prévention ou le traitement de l'alopécie; pour le traitement d'affections dermatologiques à composante immunitaire; pour le traitement d'affections du système cardiovasculaire telles que l'artériosclérose ou l'hypertension ainsi que le diabète non-insulino dépendant, pour le traitement de désordres cutanés dus à une exposition aux rayonnements U.V..

10. Composition pharmaceutique, caractérisée par le fait qu'elle comprend, dans un support pharmaceutiquement acceptable, au moins l'un des composés tels que définis à l'une quelconque des revendications 1 à 7.

11. Composition selon la revendication 10, caractérisée en ce que la concentration en composé(s) selon l'une des revendications 1 à 7 est comprise entre 0,001 % et 5 % en poids par rapport à l'ensemble de la composition.

12. Composition cosmétique, caractérisée par le fait qu'elle comprend, dans un support cosmétiquement acceptable, au moins l'un des composés tels que définis à l'une quelconque des revendications 1 à 7.

13. Composition selon la revendication 12, caractérisée en ce que la concentration en composé(s) selon l'une des revendications 1 à 7 est comprise entre 0,001 % et 3 % en poids par rapport à l'ensemble de la composition.

14. Utilisation d'une composition cosmétique telle que définie à l'une des revendications 12 ou 13 pour l'hygiène corporelle ou capillaire.

## Claims

1. Propynyl biaromatic compounds, characterized in that they correspond to the general formula (I) below: 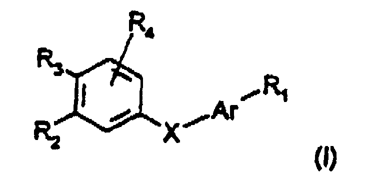 in which:
- R₁ represents
(i) the -CH₃ radical
(ii) the radical -CH₂-O-R₆
(iii) the radical -O-R₆
(iv) the radical -CO-R₇
R₆ and R₇ having the meanings given below,
- Ar represents a radical chosen from the radicals of formulae (a)-(e) below: 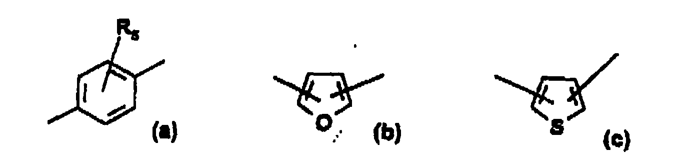 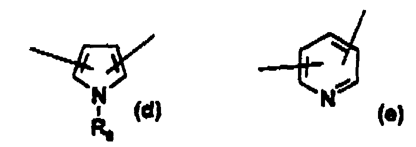 R₅ and R₆ having the meanings given below,
- X represents a radical of formula: 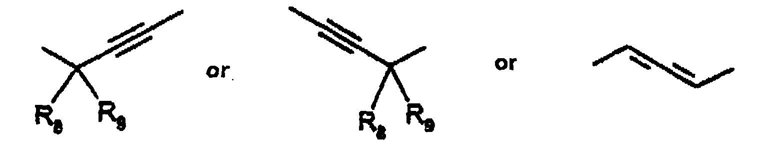 R₈ and R₉ having the meanings given below,
- R₂ and R₃, which may be identical or different, represent
(i) a hydrogen atom,
(ii) a linear or branched alkyl radical having from 1 to 20 carbon atoms,
(iii) a radical -OR₆,
(iv) a radical -SR₆,
R₆ having the meaning given below,
it being understood that R₂ and R₃, taken together, may form, with the adjacent aromatic ring, a 5- or 6-membered ring optionally substituted with methyl groups and/or optionally interrupted by an oxygen or sulphur atom,
and it being understood that R₂ and R₃ cannot simultaneously have the meanings (i), (iii) and (iv) mentioned above,
- R₄ and R₅, which may be identical or different, represent a hydrogen atom, a halogen atom, a linear or branched alkyl radical having from 1 to 20 carbon atoms or a radical -OR₆,
it being understood that when R₄ is a hydroxyl radical, then R₂ and R₃ form, with the adjacent aromatic ring, a 5- or 6-membered ring optionally substituted with methyl groups and/or optionally interrupted by an oxygen or sulphur atom,
- R₆ represents a hydrogen atom, an alkyl radical having from 1 to 12 carbon atoms or a radical -COR₁₀
R₁₀ having the meaning given below,
- R₇ represents:
(a) a hydrogen atom
(b) a lower alkyl radical
(c) a radical of formula: 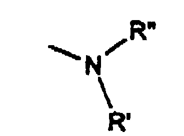
R' and R" having the meaning given below,
(d) a radical -OR₁₁,
(e) a radical -NROR₆,
R₁₁ having the meaning given below,
- R₈ and R₉, taken separately, either simultaneously have the same meaning: a hydrogen atom, or one of them represents a hydrogen atom and the other represents an alkyl radical having from 1 to 12 carbon atoms, or, taken together, form a ring -Y-(CH₂)ₙ-Y-, with Y representing an oxygen or sulphur atom and with n equal to 2 or 3,
- R₁₀ represents an alkyl radical having from 1 to 12 carbon atoms,
- R₁₁ represents a hydrogen atom, a linear or branched alkyl radical having from 1 to 20 carbon atoms, an alkenyl radical, a mono- or polyhydroxyalkyl radical, an optionally substituted aryl or aralkyl radical, a sugar residue or an amino acid or peptide residue,
- R' and R", which may be identical or different, represent a hydrogen atom, an alkyl radical having from 1 to 12 carbon atoms, a mono- or polyhydroxyalkyl radical, an optionally substituted aryl radical or an amino acid or sugar residue, or alternatively, taken together, form a heterocycle,
as well as the salts thereof and the optical and geometrical isomers thereof.

2. Compounds, characterized in that they correspond to the general formula (II) below: 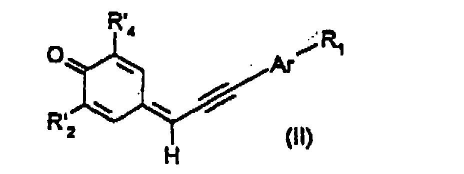 in which R₁ and Ar have the same meanings as for the general formula (I) defined in Claim 1 and R'₂ and R'₄, which may be identical or different, represent a linear or branched alkyl radical having from 1 to 20 carbon atoms.

3. Compounds according to Claim 1 or 2, characterized in that they are in the form of salts of an alkali metal or alkaline earth metal, or alternatively of zinc or of an organic amine.

4. Compounds according to Claim 1, characterized in that they are taken, alone or as mixtures, from the group consisting of:
- 4-[3-(3,5-Di-tert-butyl-4-hydroxyphenyl)-1-propynyl]benzoic acid.
- 2-Hydroxy-4-[3-(3,5-di-tert-butyl-4-hydroxyphenyl)-1-propynyl]benzoic acid.
- 4-[3-(5,6,7,8-Tetrahydro-5,5,8,8-tetramethyl-2-naphthyl)-1-propynyl]benzoic acid.
- Methyl 2-hydroxy-4-[3-(5,6,7,8-tetrahydro-5,5,8,8-tetramethyl-2-naphthyl)-1-propynyl]benzoate.
- 2-Hydroxy-4-[3-(5,6,7,8-tetrahydro-5,5,8,8-tetramethyl-2-naphthyl)-1-propynyl]benzoic acid.
- 2-Hydroxy-4-[3-(3-hydroxy-5,6,7,8-tetrahydro-5,5,8,8-tetramethyl-2-naphthyl)-1-propynyl]benzoic acid.
- 2-Hydroxy-4-[3-(5,6,7,8-tetrahydro-5,5,8,8-tetramethyl-2-naphthyl)-1-propynyl]benzenemethanol.
- Diethanolamine 2-hydroxy-4-[3-(5,6,7,8-tetrahydro-5,5,8,8-tetramethyl-2-naphthyl)-1-propynyl]benzoate.
- Lithium 2-hydroxy-4-[3-(5,6,7,8-tetrahydro-5,5,8,8-tetramethyl-2-naphthyl)-1-propynyl]benzoate.
- 4-[3-(5,6,7,8-Tetrahydro-5,5,8,8-tetramethyl-2-naphthyl) -2-propynyl]benzoic acid.
- 2-Hydroxy-4-[3-(4,4-dimethylthiochroman-6-yl)-1-propynyl]benzoic acid.
- 2-Hydroxy-4-[3-(8,8-dimethyl-5,6,7,8-tetrahydro-2-naphthyl)-1-propynyl]benzoic acid.
- 2-Hydroxy-4-[3-15,5-dimethyl-5,6,7,8-tetrahydro-2-naphthyl)-1-propynyl]benzoic acid.
- Ethyl 4-[3-(5,6,7,8-tetrahydro-5,5,8,8-tetramethyl-2-naphthyl)-1-propynyl]benzoate.
- 4-[3-(5,6,7,8-Tetrahydro-5,5,8,8-tetramethyl-2-naphthyl)-1-propynyl]benzamide.
- N-Ethyl-4-[3-(5,6,7,8-tetrahydro-5,5,8,8-tetramethyl-2-naphthyl)-1-propynyl]benzamide.
- 4-[3-(5,6,7,8-Tetrahydro-5,5,8,8-tetramethyl-2-naphthyl)-1-propynyl]benzoic acid morpholide.
- N-(4-Hydroxyphenyl)-4-[3-(5,6,7,8-tetrahydro-5,5,8,8-tetramethyl-2-naphthyl)-1-propynyl]benzamide.
- 4-[3-(5,6,7,8-Tetrahydro-5,5,8,8-tetramethyl-2-naphthyl)-1-propynyl]benzaldehyde.
- 4-[3-(5,6,7,8-Tetrahydro-5,5,8,8-tetramethyl-2-naphthyl)-1-propynyl]phenol.
- [3-(5,6,7,8-Tetrahydro-5,5,8,8-tetramethyl-naphthyl)-1-propynyl]benzenemethanol.
- 4-[3-(5,6,7,8-Tetrahydro-5,5,8,8-tetramethyl-2-naphthyl)-1-propynyl]toluene.
- Hexyl 4-[3-(5,6,7,8-tetrahydro-5,5,8,8-tetramethyl-2-naphthyl)-1-propynyl]benzoate.
- N-Hydroxy-4-[3-(5,6,7,8-tetrahydro-5,5,8,8-tetramethyl-2-naphthyl)-1-propynyl]benzamide.
- N-Hydroxy-2-hydroxy-4-[3-(5,6,7,8-tetrahydro-5,5,8,8-tetramethyl-2-naphthyl)-1-propynyl]benzamide.
- 2-Methyl-4-[3-(5,6,7,8-tetrahydro-5,5,8,8-tetramethyl-2-naphthyl)-1-propynyl]benzoic acid.
- 3-Methyl-4-[3-(5,6,7,8-tetrahydro-5,5,8,8-tetramethyl-2-naphthyl)-1-propynyl]benzoic acid.
- 6-[3-(5,5,8,8-Tetramethyl-5,6,7,8-tetrahydro-2-naphthyl)propa-1,2-dienyl]nicotinic acid.
- 4-[3-(5,5,8,8-Tetramethyl-5,6,7,8-tetrahydro-2-naphthyl)propa-l,2-dienyl]benzoic acid.
- 2-Hydroxy-4-[3-(5,5,8,8-tetramethyl-5,6,7,8-tetrahydro-2-naphthyl)propa-1,2-dienyl]benzoic acid.
- 2-Hydroxy-4-[3-(5,6,7,8-tetrahydro-5,5,8,8-tetramethyl-2-naphthyl)-1-butynyl]benzoic acid.
- 5-[3-(5,6,7,8-Tetrahydro-5,5,8,8-tetramethyl-2-naphthyl)-1-propynyl]-2-pyridinecarboxylic acid.
- 4-[3-(3,5,5,8,8-Pentamethyl-5,6,7,8-tetrahydro-2-naphthyl)-1-propynyl]benzoic acid.
- 2-[3-(5,6,7,8-Tetrahydro-5,5,8,8-tetramethyl-2-naphthyl)-1-propynyl]-4-thiophenecarboxylic acid.
- 2-[3-(3,5,5,8,8-Pentamethyl-5,6,7,8-tetrahydro-2-naphthyl)-1-propynyl]-4-thiophenecarboxylic acid.
- 2-Hydroxy-4-[3-(3-*tert*-butyl-4-methoxyphenyl)-1-propynyl]benzoic acid.
- 2-Hydroxy-4-[3-(3-*tert*-butyl-4-hydroxyphenyl)-1-propynyl]benzoic acid.

5. Compounds according to Claim 2, characterized in that they are taken, alone or as mixtures, from the group consisting of:
- 4-[3-(3,5-Di-tert-butyl-4-oxo-2,5-cyclohexadien-1-ylidene)-1-propynyl]benzoic acid.
- 2-Hydroxy-4-[3-(3,5-di-tert-butyl-4-oxo-2,5-cyclohexadien-1-ylidene)-1-propynyl]benzoic acid.

6. Compounds according to Claim 1 or 2, characterized in that they have at least one, and preferably all, of the following characteristics:
- R₁ represents the radical -CO-R₇,
- Ar represents the radicals of formula (a) or (e) .

7. Compounds according to one of Claims 1, 3 or 6, characterized in that they have a formula (I) as defined in Claim 1, in which R₈ and R₉, taken separately, either represent hydrogen atoms or one of them represents a hydrogen atom and the other represents an alkyl radical having from 1 to 12 carbon atoms.

8. Compounds of formula (I) or (II) according to any one of the preceding claims, for use as a medicinal product.

9. Compounds according to Claim 8, for use as a medicinal product intended for treating dermatological complaints associated with a keratinization disorder which has a bearing on differentiation and on proliferation, in particular for treating common acne, comedones, polymorphonuclear leukocytes, acne rosacea, nodulocystic acne, acne conglobata, senile acne and secondary acnes such as solar, medication-related or occupational acne; for treating other types of keratinization disorder, in particular ichthyosis, ichthyosiform states, Darier's disease, palmoplantar keratoderma, leucoplasias and leucoplasiform states, and cutaneous or mucous (buccal) lichen; for treating other dermatological complaints associated with a keratinization disorder with an inflammatory and/or immunoallergic component and, in particular, all forms of psoriasis, whether it is cutaneous, mucous or ungual psoriasis and even psoriatic rheumatism, or alternatively cutaneous atopy, such as eczema or respiratory atopy or alternatively gingival hypertrophy; the compounds may also be used in certain inflammatory complaints which do not exhibit a disorder of keratinization; for treating all dermal or epidermal proliferations, whether benign or malignant and whether they are of viral origin or otherwise, such as common warts, flat warts and verruciform epidermodysplasia and oral or florid papillomatoses and proliferations which can be induced by ultraviolet radiation, in particular in the case of basocellular and spinocellular epithelioma; for treating other dermatological disorders such as bullosis and collagen diseases; for treating certain ophthalmological disorders, in particular corneopathies; for repairing or combating ageing of the skin whether this is light-induced or chronological ageing, or for reducing actinic keratoses and pigmentations, or any pathologies associated with chronological or actinic ageing; for preventing or curing the stigmata of epidermal and/or dermal atrophy induced by local or systemic corticosteroids, or any other form of cutaneous atrophy, for preventing or treating cicatrization disorders or for preventing or repairing vibices; for promoting cicatrization, for combating disorders of sebaceous functioning such as the hyperseborrhoea of acne or simple seborrhoea; for treating or preventing cancerous or precancerous states, more particularly promyelocytic leukemias; for treating inflammatory complaints such as arthritis, for treating any general or skin complaint of viral origin; for preventing or treating alopecia; for treating dermatological complaints with an immune component; for treating complaints of the cardiovascular system such as arteriosclerosis or hypertension, as well as insulin-independent diabetes, and for treating skin disorders due to exposure to UV radiation.

10. Pharmaceutical composition, characterized in that it comprises, in a pharmaceutically acceptable support, at least one of the compounds as defined in any one of Claims 1 to 7.

11. Composition according to Claim 10, characterized in that the concentration of compound(s) according to one of Claims 1 to 7 is between 0.001% and 5% by weight relative to the composition as a whole.

12. Cosmetic composition, characterized in that it comprises, in a cosmetically acceptable support, at least one of the compounds as defined in any one of Claims 1 to 7.

13. Composition according to Claim 12, characterized in that the concentration of compound(s) according to one of Claims 1 to 7 is between 0.001% and 3% by weight relative to the composition as a whole.

14. Use of a cosmetic composition as defined in either of Claims 12 and 13, for body or hair hygiene.

## Patentansprüche

1. Diaromatische Propinyl-Verbindungen, dadurch gekennzeichnet, daß sie der folgenden allgemeine Formel (I) 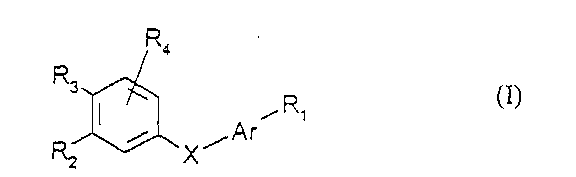 entsprechen, in der bedeuten:
- R₁
(i) die Gruppe -CH₃,
(ii) die Gruppe -CH₂-O-R₆,
(iii) die Gruppe -O-R₆,
(iv) die Gruppe -CO-R₇,
worin R₆ und R₇ die weiter unten angegebene Bedeutung haben,
- Ar eine Gruppe, die unter den Gruppen der folgenden Formeln (a)-(e) 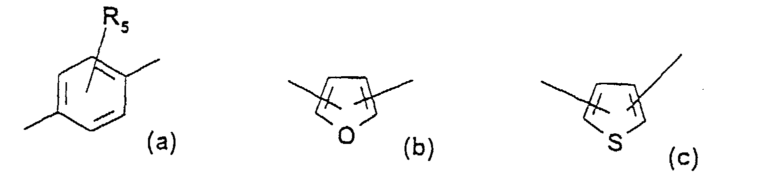 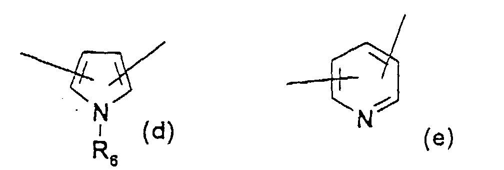 ausgewählt ist, worin R₆ und R₇ die weiter unten angegebene Bedeutung haben,
- X eine Gruppe der Formel 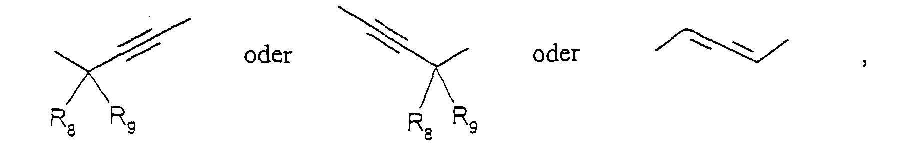 worin R₈ und R₉ die weiter unten angegebene Bedeutung haben,
- R₂ und R₃, die gleich oder verschieden sind,
(i) ein Wasserstoffatom
(ii) eine geradkettige oder verzweigte Alkylgruppe mit 1 bis 20 Kohlenstoffatomen,
(iii) eine Gruppe -OR₆,
(iv) eine Gruppe -SR₆,
worin R₆ die weiter unten angegebene Bedeutung hat,
mit der Maßgabe, daß R₂ und R₃ zusammen genommen mit dem benachbarten aromatischen Ring einen Ring mit 5 oder 6 Kettengliedern bilden können, der gegebenenfalls mit Methylgruppen substituiert ist und/oder gegebenenfalls durch ein Sauerstoffatom oder Schwefelatom unterbrochen ist, und
mit der Maßgabe, daß R₂ und R₃ nicht gleichzeitig die oben angegebenen Bedeutungen (i), (iii) und (iv) haben können,
- R₄ und R₅, die gleich oder verschieden sind, ein Wasserstoffatom, ein Halogenatom, eine geradkettige oder verzweigte Alkylgruppe, die 1 bis 20 Kohlenstoffatome aufweist, eine Gruppe -OR₆,
mit der Maßgabe, daß, wenn R₄ eine Hydroxygruppe darstellt, R₂ und R₃ mit dem benachbarten aromatischen Ring einen Ring mit 5 oder 6 Kettengliedern bilden, der gegebenenfalls mit Methylgruppen substituiert ist und/oder gegebenenfalls durch ein Sauerstoffatom oder Schwefelatom unterbrochen ist,
- R₆ ein Wasserstoffatom, eine Alkylgruppe mit 1 bis 12 Kohlenstoffatomen oder eine Gruppe -COR₁₀, worin R₁₀ die weiter unten angegebene Bedeutung hat,
- R₇
(a) ein Wasserstoffatom
(b) eine niedere Alkylgruppe,
(c) eine Gruppe der Formel 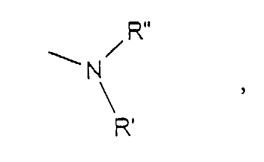 worin R' und R" die weiter unten angegebene Bedeutung haben,
(d) eine Gruppe -OR₁₁, worin R₁₁ die weiter unten angegebene Bedeutung hat,
(e) eine Gruppe -NROR₆,
- R₈ und R₉ Gruppen, die einzeln genommen entweder gleichzeitig Wasserstoff bedeuten oder von denen die eine Gruppe Wasserstoff und die andere Gruppe eine Alkylgruppe mit 1 bis 12 Kohlenstoffatomen darstellt oder die zusammen genommen einen Ring -Y-(CH₂)ₙ-Y- bilden, worin Y ein Sauerstoff- oder Schwefelatom bedeutet und n die Zahl 2 oder 3 ist,
- R₁₀ eine niedere Alkylgruppe,
- R₁₁ ein Wasserstoffatom, eine-geradkettige oder verzweigte Alkylgruppe mit 1 bis 20 Kohlenstoffatomen, Alkenyl, Mono- oder Polyhydroxyalkyl, Aryl oder Aralkyl, das/die gegebenenfalls substituiert ist/sind, oder ein Zuckerrest, Aminosäurerest oder Peptidrest,
- R' und R", die gleich oder verschieden sind, ein Wasserstoffatom, eine Alkylgruppe mit 1 bis 12 Kohlenstoffatomen, Mono- oder Polyhydroxyalkyl, Aryl, das gegebenenfalls substituiert ist, oder ein Aminosäurerest oder ein Zuckerrest oder Reste, die zusammen genommen einen Heterocyclus bilden.

2. Verbindungen, dadurch gekennzeichnet, daß sie der folgenden allgemeinen Formel (II) 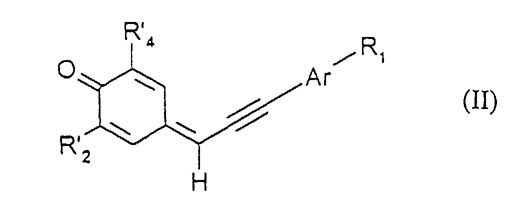 entsprechen, in der R₁ und Ar die gleiche Bedeutung wie in der in Anspruch 1 definierten allgemeinen Formel (I) haben und R'₂ und R'₄, die gleich oder verschieden sind, geradkettiges oder verzweigtes Alkyl mit 1 bis 20 Kohlenstoffatomen bedeuten.

3. Verbindungen nach Anspruch 1 oder 2, dadurch gekennzeichent, daß sie in Form von Alkalimetallsalzen, Erdalkalimetallsalzen, Zinksalzen oder Salzen eines organischen Amins vorliegen.

4. Verbindungen nach Anspruch 1, dadurch gekennzeichnet, daß sie einzeln oder als Gemische ausgewählt werden unter:
- 4-[3-(3,5-Di-*tert*.-butyl-4-hydroxyphenyl)-1-propinyl]-benzoesäure,
- 2-Hydroxy-4-[3-(3,5-di-*tert*.-butyl-4-hydroxyphenyl)-1-propinyl]-benzoesäure,
- 4-[3-(5,6,7,8-Tetrahydro-5,5,8,8-tetramethyl-2-naphthyl)-1-propinyl]-benzoesäure,
- 2-Hydroxy-4-[3-(5,6,7,8-tetrahydro-5,5,8,8-tetramethyl-2-naphthyl)-1-propinyl]-benzoesäuremethylester,
- 2-Hydroxy-4-[3-(5,6,7,8-tetrahydro-5,5,8,8-tetramethyl-2-naphthyl)-1-propinyl]-benzoesäure,
- 2-Hydroxy-4-[3-(3-Hydroxy-5,6,7,8-tetrahydro-5,5,8,8-tetramethyl-2-naphthyl)-1-propinyl]-benzoesäure,
- 2-Hydroxy-4-[3-(5,6,7,8-tetrahydro-5,5,8,8-tetramethyl-2-naphthyl)-1-propinyl]-benzolmethanol,
- 2-Hydroxy-4-[3-(5,6,7,8-tetrahydro-5,5,8,8-tetramethyl-2-naphthyl)-1-propinyl]-benzoesäurediethanolaminester,
- Lithium-2-hydroxy-4-[3-(5,6,7,8-tetrahydro-5,5,8,8-tetramethyl-2-naphthyl)-1-propinyl]-benzoat,
- 4-[3-(5,6,7,8-tetrahydro-5,5,8,8-tetramethyl-2-naphthyl)-1-propinyl]-benzoesäure,
- 2-Hydroxy-4-[3-(4,4-dimethylthiochroman-6-yl)-1-propinyl]-benzoesäure,
- 2-Hydroxy-4-[3-(8,8-dimethyl-5,6,7,8-tetrahydro-2-naphthyl)-1-propinyl]-benzoe-säure,
- 2-Hydroxy-4-[3-(5,5-dimethyl-5,6,7,8-tetrahydro-2-naphthyl)-1-propinyl]-benzoe-säure,
- 4-[3-(5,6,7,8-Tetrahydro-5,5,8,8-tetramethyl-2-naphthyl)-1-propinyl]-benzoesäure-ethylester,
- 4-[3-(5,6,7,8-Tetrahydro-5,5,8,8-tetramethyl-2-naphthyl)-1-propinyl]-benzamid,
- N-Ethyl-4-[3-(5,6,7,8-tetrahydro-5,5,8,8-tetramethyl-2-naphthyl)-1-propinyl]-benz-amid,
- Morpholid der 4-[3-(5,6,7,8-Tetrahydro-5,5,8,8-tetramethyl-2-naphthyl)-1-propi-nyl]-benzoesäure,
- N-(4-Hydroxyphenyl)-4-[3-(5,6,7,8-tetrahydro-5,5,8,8-tetramethyl-2-naphthyl)-1-propinyl]-benzamid,
- 4-[3-(5,6,7,8-Tetrahydro-5,5,8,8-tetramethyl-2-naphthyl)-1-propinyl]-benzaldehyd,
- 4-[3-(5,6,7,8-Tetrahydro-5,5,8,8-tetramethyl-2-naphthyl)-1-propinyl]-phenol,
- 4-[3-(5,6,7,8-Tetrahydro-5,5,8,8-tetramethyl-2-naphthyl)-1-propinyl]-benzolmethanol.
- 4-[3-(5,6,7,8-Tetrahydro-5,5,8,8-tetramethyl-2-naphthyl)-1-propinyl]-toluol,
- 4-[3-(5,6,7,8-Tetrahydro-5,5,8,8-tetramethyl-2-naphthyl)-1-propinyl]-benzoesäurehexylester,
- N-Hydroxy-4-[3-(5,6,7,8-tetrahydro-5,5,8,8-tetramethyl-2-naphthyl)-1-propinyl]-benzamid,
- N-Hydroxy-2-hydroxy-4-[3-(5,6,7,8-tetrahydro-5,5,8,8-tetramethyl-2-naphthyl)-1-propinyl]-benzamid,
- 2-Methyl-4-[3-(5,6,7,8-tetrahydro-5,5,8,8-tetramethyl-2-naphthyl)-1-propinyl]-benzoesäure,
- 3-Methyl-4-[3-(5,6,7,8-tetrahydro-5,5,8,8-tetramethyl-2-naphthyl)-1-propinyl]-benzoesäure,
- 6-[3-(5,5,8,8-Tetramethyl-5,6,7,8-tetrahydro-2-naphthyl)-propa-1,2-dienyl]-nicotinsäure,
- 4-[3-(5,5,8,8-Tetramethyl-5,6,7,8-tetrahydro-2-naphthyl)-propa-1,2-dienyl]-benzoesäure,
- 2-Hydroxy-4-[3-(5,5,8,8-tetramethyl-5,6,7,8-tetrahydro-2-naphthyl)-propa-1,2-dienyl]-benzoesäure,
- 2-Hydroxy-4-[3-(5,6,7,8-tetrahydro-5,5,8,8-tetramethyl-2-naphthyl)-1-butinyl]-benzoesäure,
- 5-[3-(5,6,7,8-Tetrahydro-5,5,8,8-tetramethyl-2-naphthyl)-1-propinyl]-2-pyridincarbonsäure,
- 4-[3-(3,5,5,8,8-Pentamethyl-5,6,7,8-tetrahydro-2-naphthyl)-1-propinyl]-benzoesäure,
- 2-[3-(5,6,7,8-Tetrahydro-5,5,8,8-tetramethyl-2-naphthyl)-1-propinyl]-4-thiophencarbonsäure,
- 2-[3-(3,5,5,8,8-Pentamethyl-5,6,7,8-tetrahydro-2-naphthyl)-1-propinyl]-4-thiophencarbonsäure,
- 2-Hydroxy-4-[3-(3-*tert*.-butyl-4-methoxyphenyl)-1-propinyl]-benzoesäure,
- 2-Hydroxy-4-[3-(3-*tert*.-butyl-4-hydroxyphenyl)-1-propinyl]-benzoesäure.

5. Verbindungen nach Anspruch 2, dadurch gekennzeichnet, daß sie einzeln oder im Gemisch ausgewählt werden unter
- 4-[3-(3,5-Di-*tert*.-butyl-4-oxo-2,5-cyclohexadien-1-yliden)-1-propinyl]-benzoesäure,
- 2-Hydroxy-4-[3-(3,5-di-*tert*.-butyl-4-oxo-2,5-cyclohexadien-1-yliden)-1-propinyl]-benzoesäure.

6. Verbindungen nach Anspruch 1 oder 2, dadurch gekennzeichnet, daß sie mindestens eine der beiden und vorzugsweise alle folgende Bedingungen erfüllen:
- R₁ stellt die Gruppe -CO-R₇ dar,
- Ar stellt die Gruppen der Formel (a) oder (e) dar.

7. Verbindungen nach einem der Ansprüche 1, 3 oder 6, dadurch gekennzeichnet, daß sie eine wie in Anspruch 1 definierte Formel (I) aufweisen, in der R₈ und R₉ einzeln genommen entweder Wasserstoffatome bedeuten oder von denen der eine Rest Wasserstoff und der andere Rest eine Alkylgruppe mit 1 bis 12 Kohlenstoffatomen bedeutet.

8. Verbindungen der Formel (I) oder (II) nach einem der vorhergehenden Ansprüche für eine Verwendung als Arzneimittel.

9. Verbindungen nach Anspruch 8 zur Verwendung als Arzneimittel, das vorgesehen ist für die Behandlung von dermatologischen Erkrankungen, die mit Störungen der Keratinisierung verbunden sind, die die Differenzierung und die Vermehrung betreffen, insbesondere für die Behandlung der Akne vulgaris, Akne comedonica, der polymorphen Akne, der Akne rosaceae, der Akne nodularis, der Akne conglobata, der Altersakne, der sekundären Aknen, wie der Akne solaris, der Medikamentenakne, oder der Akne professionalis, für die Behandlung von anderen Typen von Störungen der Keratinisierung, insbesondere der Ichtyosen, der ichtyosoformen Zustände, der Darier-Krankheit, der palmoplantaren Keratose, der Leukoplasien und der leukoplasieartigen Zustände, der Hautflechten und Schleimhautflechten (bukkal), für die Behandlung anderer dermatologischer Erkrankungen, die mit einer Störung der Keratinisierung verbunden sind mit einem entzündlichen und/oder immunoallergischen Anteil, und insbesondere für die Behandlung aller Arten von Psoriasis, die die Haut, die Schleimhäute und die Finger- und Zehennägel betreffen, und auch des psoriatischen Rheumas, und von Überempfindlichkeiten der Haut, wie Ekzema, respiratorischer Überempfindlichkeit, Hypertrophie des Zahnfleisches, wobei die Verbindungen ferner bei einigen Entzündungen verwendet werden können, die nicht mit Störungen der Keratinisierung zusammenhängen, für die Behandlung jeder Art von Wucherungen der Dermis und der Epidermis, die gutartig oder bösartig sind, virusbedingt oder nicht virusbedingt sind, wie Verruca vulgaris, Verruca plana, Epidermodysplasia verruciformis, die orale Papillomatose, die Papillomatosis florida, und für die Behandlung von Wucherungen, die durch UV-Strahlung hervorgerufen werden können, insbesondere Epithelioma basocellulare und spinocellulare, für die Behandlung anderer dermatologischer Störungen, wie Dermatosis bullosa und Kollagenosen, für die Behandlung einiger ophthalmologischer Erkrankungen, insbesondere der Hornhauterkrankungen, für die Behebung oder Bekämpfung der Hautalterung, und zwar sowohl der lichtbedingten als auch der mit dem Altern auftretenden Hautalterung, oder zur Verminderung der Pigmentierung und der Keratosis actinica, und für die Behandlung aller sonstigen Erkrankungen, die mit der alterungsbedingten oder lichtbedingten Alterung zusammenhängen, für die Vermeidung oder Heilung der Narben der Atrophie der Epidermis und/oder Dermis, die durch die lokale oder systemische Anwendung von Corticosteroiden hervorgerufen wird, oder jeder sonstigen Form der Hautatrophie, für die Vermeidung oder Behandlung von Störungen bei der Wundheilung oder die Vermeidung oder Behebung von Schwangerschaftsstreifen, für die Förderung der Wundheilung, zur Bekämpfung von Störungen der Talgproduktion, wie Aknehyperseborrhö oder einfache Seborrhö, bei der Behandlung von oder der Vorbeugung vor krebsartigen Zuständen oder Krebsvorstufen, insbesondere der promyelocytären Leukämien, bei der Behandlung von entzündlichen Krankheiten, wie Arthritis, bei der Behandlung jeder virusbedingten Erkrankung im Bereich der Haut oder an sonstigen Körperteilen, wie dem Kaposi-Syndrom, bei der Vorbeugung vor oder der Behandlung der Alopecie, bei der Behandlung von dermatologischen Erkrankungen mit immunologischem Anteil, bei der Behandlung von Erkrankungen des Herz-Kreislauf-Systems, wie der Arteriosklerose oder des Bluthochdrucks, sowie des nicht insulinabhängigen Diabetes, bei der Behandlung von Veränderungen der Haut, die durch Bestrahlung mit UV-Strahlung verursacht werden.

10. Pharmazeutische Zusammensetzung, dadurch gekennzeichnet, daß sie in einem pharmazeutisch akzeptablen Träger mindestens eine der Verbindungen, die wie in einem der Ansprüche 1 bis 7 definiert sind, enthält.

11. Zusammensetzung nach Anspruch 10, dadurch gekennzeichnet, daß die Konzentration der Verbindung(en) nach einem der Ansprüche 1 bis 7 im Bereich von 0,001 bis 5 Gew.-%, bezogen auf die gesamte Zusammensetzung, liegt.

12. Kosmetische Zusammensetzung, dadurch gekennzeichnet, daß sie in einem kosmetisch akzeptablen Träger mindestens eine der Verbindungen, die wie in einem der Ansprüche 1 bis 7 definiert sind; enthält.

13. Zusammensetzung nach Anspruch 12, dadurch gekennzeichnet, daß die Konzentration der Verbindung(en) nach einem der Ansprüche 1 bis 7 im Bereich von 0,001 bis 3 Gew.-%, bezogen auf die gesamte Zusammensetzung, liegt.

14. Verwendung einer kosmetischen Zusammensetzung, die wie in einem der Ansprüche 12 oder 13 definiert ist, für die Körperpflege oder die Haarpflege.
